# EUROPEAN PATENT APPLICATION

(11) **EP 3 777 889 A1**
(43) Date of publication of application: **17.02.2021**
(21) Application number: 19778158.6
(22) Date of filing: 26.03.2019
(51) Int. Cl.: A61K 39/395, A61P 35/00

(54) **USE OF PROBDNF REGULATOR IN B CELL-RELATED DISEASES**

(30) Priority: 26.03.2018 CN 201810254609
(71) Applicant: Shanghai Yile Biotechnology Co., Ltd., Shanghai 200231 (CN)
(72) Inventor: DAI, Ruping, Shanghai 200231 (CN); CAI, Xiumei, Shanghai 200231 (CN); WANG, Huamao, Shanghai 200231 (CN)
(74) Representative: Lavoix
(86) International application number: PCT/CN2019/079735
(87) International publication number: WO 2019/184920

(57) **Abstract**

Disclosed is a use of a proBDNF regulator in B cell-related diseases. Specifically, disclosed is a use of a reagent which suppresses or activates proBDNF activity or proBDNF signaling pathways in preparing a regulator which regulates B lymphocyte function. Also disclosed is a use of a regulator in preparing a drug which treats B lymphocyte dysfunction-related diseases, a use of an antibody in preparing a reagent or reagent kit which detects/treats B lymphocyte dysfunction-related diseases, and a method for predicting a treatment or prevention effect of treating or preventing B lymphocyte dysfunction-related diseases.

Sequence Listing

<110> Shanghai Yile Biotechnology CO., LTD.

<120> USE OF PROBDNF REGULATOR IN B CELL-RELATED DISEASES

<130> BET 20M3589

<140> EP 19778158.6

<141> 2019-03-26

<150> CN 201810254609.X
<151> 2018-03-26

<160> 38

<170> SIPOSequenceListing 1.0

<210> 1
<211> 330
<212> DNA
<213> Homo sapiens

<220>
<223> proBDNF

<400> 1
gcccccatga aagaagcaaa catccgagga caaggtggct tggcctaccc aggtgtgcgg 60
acccatggga ctctggagag cgtgaatggg cccaaggcag gttcaagagg cttgacatca 120
ttggctgaca ctttcgaaca catgatagaa gagctgttgg atgaggacca gaaagttcgg 180
cccaatgaag aaaacaataa ggacgcagac ttgtacacgt ccagggtgat gctcagtagt 240
caagtgcctt tggagcctcc tcttctcttt ctgctggagg aatacaaaaa ttacctagat 300
gctgcaaaca tgtccatgag ggtccggcgc 340

<210> 2
<211> 110
<212> PRT
<213> Homo sapiens

<220>
<223> precursor domain of proBDNF

<400> 2
Ala Pro Met Lys Glu Ala Asn Ile Arg Gly Gln Gly Gly Leu Ala Tyr
1               5                   10                  15
Pro Gly Val Arg Thr His Gly Thr Leu Glu Ser Val Asn Gly Pro Lys
            20                  25                  30
Ala Gly Ser Arg Gly Leu Thr Ser Leu Ala Asp Thr Phe Glu His Val
        35                  40                  45
Ile Glu Glu Leu Leu Asp Glu Asp Gln Lys Val Arg Pro Asn Glu Glu
    50                  55                  60
Asn Asn Lys Asp Ala Asp Leu Tyr Thr Ser Arg Val Met Leu Ser Ser
65                  70                  75                  80
Gln Val Pro Leu Glu Pro Pro Leu Leu Phe Leu Leu Glu Glu Tyr Lys
                85                  90                  95
Asn Tyr Leu Asp Ala Ala Asn Met Ser Met Arg Val Arg Arg
            100                 105                 110

<210> 3
<211> 5

<212>  PRT
<213>  Artificial Sequence

<220>
<223>  HCDR1 of 1D3


<400>  3
Gly Tyr Asp Met His
1               5

<210>  4
<211>  16
<212>  PRT
<213>  Artificial Sequence

<220>
<223>  HCDR2 of 1D3


<400>  4
Gly Leu Gly Met Glu Gly Asp Ser Tyr Tyr Ser Ala Ser Val Lys Gly
1               5                   10                  15

<210>  5
<211>  7
<212>  PRT
<213>  Artificial Sequence

<220>
<223>  HCDR3 of 1D3


<400>  5
Asp Val His Gly Phe Asp Val
1               5

<210>  6
<211>  16
<212>  PRT
<213>  Artificial Sequence

<220>
<223>  LCDR1 of 1D3



<400>  6
Arg Ser Ser Gln Ser Leu Leu Tyr Ser Asn Gly Tyr Thr Tyr Leu Asp
1               5                   10                  15

<210>  7
<211>  7
<212>  PRT
<213>  Artificial Sequence

<220>
<223>  LCDR2 of 1D3



<400>  7
Met Gly Ser Asn Arg Ala Ser

1                5

<210>  8
<211>  9
<212>  PRT
<213>  Artificial Sequence

<220>
<223>  LCDR3of 1D3

<400>  8
Met Gln Ala Leu Gln Thr Pro Leu Thr
1               5

<210>  9
<211>  115
<212>  PRT
<213>  Artificial Sequence

<220>
<223>  VH of 1D3

<400>  9
Gln Val Gln Leu Val Glu Ser Gly Gly Gly Leu Ile Gln Pro Gly Gly
1               5                   10                  15
Ser Met Arg Leu Ser Cys Ala Ala Ser Gly Phe Ser Leu Ser Gly Tyr
            20                  25                  30
Asp Met His Trp Val Arg Gln Ile Ala Gly Lys Gly Leu Glu Trp Val
        35                  40                  45
Ala Gly Leu Gly Met Glu Gly Asp Ser Tyr Tyr Ser Ala Ser Val Lys
    50                  55                  60
Gly Arg Phe Thr Ile Ser Arg Gln Asp Ala Lys Asn Ser Leu Tyr Leu
65                  70                  75                  80
Glu Met Lys Asp Leu Gly Gly Gly Asp Thr Ala Val Tyr Tyr Cys Leu
                85                  90                  95
Arg Asp Val His Gly Phe Asp Val Trp Gly Gln Gly Thr Thr Val Thr
            100                 105                 110
Val Ser Ser
        115

<210>  10
<211>  113
<212>  PRT
<213>  Artificial Sequence

<220>
<223>  VL of 1D3

<400>  10
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu Tyr Ser
            20                  25                  30
Asn Gly Tyr Thr Tyr Leu Asp Trp Tyr Leu Gln Arg Pro Gly Gln Ser
        35                  40                  45
Pro Gln Leu Leu Ile Tyr Met Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Leu Glu Ile Lys
            100                 105                 110
Arg

<210>  11
<211>  442
<212>  PRT
<213>  Artificial Sequence

<220>
<223>  heavy chain of 1D3

<400>  11
Gln Val Gln Leu Val Glu Ser Gly Gly Gly Leu Ile Gln Pro Gly Gly
1               5                   10                  15
Ser Met Arg Leu Ser Cys Ala Ala Ser Gly Phe Ser Leu Ser Gly Tyr
            20                  25                  30
Asp Met His Trp Val Arg Gln Ile Ala Gly Lys Gly Leu Glu Trp Val
        35                  40                  45
Ala Gly Leu Gly Met Glu Gly Asp Ser Tyr Tyr Ser Ala Ser Val Lys
    50                  55                  60
Gly Arg Phe Thr Ile Ser Arg Gln Asp Ala Lys Asn Ser Leu Tyr Leu
65                  70                  75                  80
Glu Met Lys Asp Leu Gly Gly Gly Asp Thr Ala Val Tyr Tyr Cys Leu
                85                  90                  95
Arg Asp Val His Gly Phe Asp Val Trp Gly Gln Gly Thr Thr Val Thr
            100                 105                 110
Val Ser Ser Ala Ser Thr Lys Gly Pro Ser Val Phe Pro Leu Ala Pro
        115                 120                 125
Cys Ser Arg Ser Thr Ser Glu Ser Thr Ala Ala Leu Gly Cys Leu Val
    130                 135                 140
Lys Asp Tyr Phe Pro Glu Pro Val Thr Val Ser Trp Asn Ser Gly Ala
145                 150                 155                 160
Leu Thr Ser Gly Val His Thr Phe Pro Ala Val Leu Gln Ser Ser Gly
                165                 170                 175
Leu Tyr Ser Leu Ser Ser Val Val Thr Val Pro Ser Ser Ser Leu Gly
            180                 185                 190
Thr Lys Thr Tyr Thr Cys Asn Val Asp His Lys Pro Ser Asn Thr Lys
        195                 200                 205
Val Asp Lys Arg Val Glu Ser Lys Tyr Gly Pro Pro Cys Pro Ser Cys
    210                 215                 220
Pro Ala Pro Glu Phe Leu Gly Gly Pro Ser Val Phe Leu Phe Pro Pro
225                 230                 235                 240
Lys Pro Lys Asp Thr Leu Met Ile Ser Arg Thr Pro Glu Val Thr Cys
                245                 250                 255
Val Val Val Asp Val Ser Gln Glu Asp Pro Glu Val Gln Phe Asn Trp
            260                 265                 270
Tyr Val Asp Gly Val Glu Val His Asn Ala Lys Thr Lys Pro Arg Glu
        275                 280                 285
Glu Gln Phe Asn Ser Thr Tyr Arg Val Val Ser Val Leu Thr Val Leu
    290                 295                 300
His Gln Asp Trp Leu Asn Gly Lys Glu Tyr Lys Cys Lys Val Ser Asn
305                 310                 315                 320
Lys Gly Leu Pro Ser Ser Ile Glu Lys Thr Ile Ser Lys Ala Lys Gly
                325                 330                 335
Gln Pro Arg Glu Pro Gln Val Tyr Thr Leu Pro Pro Ser Gln Glu Glu

                    340                         345                         350
Met Thr Lys Asn Gln Val Ser Leu Thr Cys Leu Val Lys Gly Phe Tyr
            355                         360                         365
Pro Ser Asp Ile Ala Val Glu Trp Glu Ser Asn Gly Gln Pro Glu Asn
        370                         375                         380
Asn Tyr Lys Thr Thr Pro Pro Val Leu Asp Ser Asp Gly Ser Phe Phe
385                         390                         395                         400
Leu Tyr Ser Arg Leu Thr Val Asp Lys Ser Arg Trp Gln Glu Gly Asn
                        405                         410                         415
Val Phe Ser Cys Ser Val Met His Glu Ala Leu His Asn His Tyr Thr
                420                         425                         430
Gln Lys Ser Leu Ser Leu Ser Pro Gly Lys
            435                         440

<210>  12
<211>  219
<212>  PRT
<213>  Artificial Sequence

<220>
<223>  Light chain of 1D3

<400>  12
Asp Val Val Met Thr Gln Ser Pro Leu Ser Leu Pro Val Thr Pro Gly
1                   5                   10                  15
Glu Pro Ala Ser Ile Ser Cys Arg Ser Ser Gln Ser Leu Leu Tyr Ser
                20                  25                  30
Asn Gly Tyr Thr Tyr Leu Asp Trp Tyr Leu Gln Arg Pro Gly Gln Ser
            35                  40                  45
Pro Gln Leu Leu Ile Tyr Met Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Leu Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys
    210                 215

<210>  13
<211>  5
<212>  PRT
<213>  Artificial Sequence

<220>
<223>  HCDR1 of 2H8

<400>  13
Ser Tyr Gly Met His
1               5

<210>  14
<211>  17
<212>  PRT
<213>  Artificial Sequence

<220>
<223>  HCDR2 of 2H8

<400>  14
Val Ile Ser Gly Ser Gly Asp Ser Thr Tyr Tyr Ala Glu Ser Val Lys
1               5                   10                  15
Gly

<210>  15
<211>  10
<212>  PRT
<213>  Artificial Sequence

<400>  15
Gly Ile Leu Thr Gly Tyr Val Phe Asp Tyr
1               5                   10

<210>  16
<211>  16
<212>  PRT
<213>  Artificial Sequence

<220>
<223>  LCDR1 of 2H8

<400>  16
Arg Ser Ser Gln Ser Leu Val Ser Asn Asp Gly Asn Thr Tyr Leu Asn
1               5                   10                  15

<210>  17
<211>  7
<212>  PRT
<213>  Artificial Sequence

<220>
<223>  LCDR2 of 2H8

<400>  17
Met Val Ser Lys Trp Asp Ser
1               5

<210>  18
<211>  9
<212>  PRT
<213>  Artificial Sequence

<220>
<223>  LCDR3 of 2H8

<400>  18
Met Gln Ser Thr His Trp Pro Pro Thr
1               5

<210>  19
<211>  119
<212>  PRT
<213>  Artificial Sequence

<220>
<223>  VH of 2H8

<400>  19
Glu Val Gln Leu Val Glu Thr Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ser Val Ile Ser Gly Ser Gly Asp Ser Thr Tyr Tyr Ala Glu Ser Val
    50                  55                  60
Lys Gly Arg Phe Thr Ile Ser Arg Asp Asn Ala Arg Asn Thr Val Tyr
65                  70                  75                  80
Leu Gln Met Asn Ser Leu Arg Ala Glu Asp Thr Ala Val Tyr Tyr Cys
                85                  90                  95
Ala Ser Gly Ile Leu Thr Gly Tyr Val Phe Asp Tyr Trp Gly Lys Gly
            100                 105                 110
Thr Met Val Thr Val Ser Ser
        115

<210>  20
<211>  113
<212>  PRT
<213>  Artificial Sequence

<220>
<223>  VL of 2H8

<400>  20
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Ser Asn
            20                  25                  30
Asp Gly Asn Thr Tyr Leu Asn Trp Phe Gln Gln Arg Pro Gly Gln Pro
        35                  40                  45
Pro Arg Arg Leu Ile Tyr Met Val Ser Lys Trp Asp Ser Gly Val Pro
    50                  55                  60
Asp Arg Phe Ser Gly Ser Gly Ser Gly Thr Asp Phe Thr Leu Arg Ile
65                  70                  75                  80
Ser Arg Val Glu Ala Glu Asp Val Gly Val Tyr Tyr Cys Met Gln Ser
                85                  90                  95
Thr His Trp Pro Pro Thr Phe Gly Gly Gly Thr Lys Leu Glu Ile Lys
            100                 105                 110
Arg

<210> 21
<211> 446
<212> PRT
<213> Artificial Sequence

<220>
<223> heavy chain of 2H8

<400> 21
Glu Val Gln Leu Val Glu Thr Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ser Val Ile Ser Gly Ser Gly Asp Ser Thr Tyr Tyr Ala Glu Ser Val
    50                  55                  60
Lys Gly Arg Phe Thr Ile Ser Arg Asp Asn Ala Arg Asn Thr Val Tyr
65                  70                  75                  80
Leu Gln Met Asn Ser Leu Arg Ala Glu Asp Thr Ala Val Tyr Tyr Cys
                85                  90                  95
Ala Ser Gly Ile Leu Thr Gly Tyr Val Phe Asp Tyr Trp Gly Lys Gly
            100                 105                 110
Thr Met Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Ser Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210>  22
<211>  219
<212>  PRT
<213>  Artificial Sequence

<220>
<223>  light chain of 2H8

<400>  22
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Ser Asn
            20                  25                  30
Asp Gly Asn Thr Tyr Leu Asn Trp Phe Gln Gln Arg Pro Gly Gln Pro
        35                  40                  45
Pro Arg Arg Leu Ile Tyr Met Val Ser Lys Trp Asp Ser Gly Val Pro
    50                  55                  60
Asp Arg Phe Ser Gly Ser Gly Ser Gly Thr Asp Phe Thr Leu Arg Ile
65                  70                  75                  80
Ser Arg Val Glu Ala Glu Asp Val Gly Val Tyr Tyr Cys Met Gln Ser
                85                  90                  95
Thr His Trp Pro Pro Thr Phe Gly Gly Gly Thr Lys Leu Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys
    210                 215

<210>  23
<211>  243
<212>  PRT
<213>  Artificial Sequence

<220>
<223>  scFv of 1D3

<400>  23
Gln Val Gln Leu Val Glu Ser Gly Gly Gly Leu Ile Gln Pro Gly Gly
1               5                   10                  15
Ser Met Arg Leu Ser Cys Ala Ala Ser Gly Phe Ser Leu Ser Gly Tyr
            20                  25                  30
Asp Met His Trp Val Arg Gln Ile Ala Gly Lys Gly Leu Glu Trp Val

35                  40                  45
Ala Gly Leu Gly Met Glu Gly Asp Ser Tyr Tyr Ser Ala Ser Val Lys
    50                  55                  60
Gly Arg Phe Thr Ile Ser Arg Gln Asp Ala Lys Asn Ser Leu Tyr Leu
65                  70                  75                  80
Glu Met Lys Asp Leu Gly Gly Gly Asp Thr Ala Val Tyr Tyr Cys Leu
                85                  90                  95
Arg Asp Val His Gly Phe Asp Val Trp Gly Gln Gly Thr Thr Val Thr
            100                 105                 110
Val Ser Ser Gly Gly Gly Gly Ser Gly Gly Gly Gly Ser Gly Gly Gly
        115                 120                 125
Gly Ser Asp Val Val Met Thr Gln Ser Pro Leu Ser Leu Pro Val Thr
    130                 135                 140
Pro Gly Glu Pro Ala Ser Ile Ser Cys Arg Ser Ser Gln Ser Leu Leu
145                 150                 155                 160
Tyr Ser Asn Gly Tyr Thr Tyr Leu Asp Trp Tyr Leu Gln Arg Pro Gly
                165                 170                 175
Gln Ser Pro Gln Leu Leu Ile Tyr Met Gly Ser Asn Arg Ala Ser Gly
            180                 185                 190
Val Pro Asp Arg Phe Ser Gly Ser Gly Ser Gly Thr Asp Phe Thr Leu
        195                 200                 205
Lys Ile Ser Arg Val Glu Ala Glu Asp Val Gly Val Tyr Tyr Cys Met
    210                 215                 220
Gln Ala Leu Gln Thr Pro Leu Thr Phe Gly Gly Gly Thr Lys Leu Glu
225                 230                 235                 240
Ile Lys Arg

<210>  24
<211>  729
<212>  DNA
<213>  Artificial Sequence

<220>
<223>  Nucleotide sequence of 1D3's scFv

<400>  24
caggtgcagc tggtggagtc tgggggaggc ctgatacagc cgggggggtc gatgagactc      60
tcctgtgcag cctctggatt cagcctcagt ggatatgaca tgcactgggt ccgccaaatt     120
gcgggaaaag gtctggagtg ggtcgccggt cttgggatgg aaggtgactc atattattca     180
gcctccgtga agggccgatt caccatctcc agacaagatg ccaagaattc cctgtatctt     240
gaaatgaagg acctgggagg cggggacacg gctgtctatt actgtctaag agatgtccac     300
ggattcgacg tctggggcca agggaccacg gtcaccgtct cgagtggtgg aggcggttca     360
ggcggaggtg gttctggcgg tggcggatcg gatgttgtga tgactcagtc tccactctcc     420
ctgcccgtca cccctggaga gccggcctcc atctcctgca ggtctagtca gagcctcctg     480
tatagtaatg gatacaccta tttggattgg tacctgcaga ggccagggca gtctccacag     540
ctcctgatct atatgggttc taatcgggcc tccggggtcc ctgacaggtt cagtggcagt     600
ggatcaggca cagattttac actgaaaatc agcagagtgg aggctgagga tgttggggtt     660
tattactgca tgcaagctct acaaactccc ctcactttcg gcggagggac caagctggag     720
atcaaacgt                                                             753

<210>  25
<211>  247
<212>  PRT
<213>  Artificial Sequence

<220>
<223>  scFv of 2H8

<400>  25
Glu Val Gln Leu Val Glu Thr Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ser Val Ile Ser Gly Ser Gly Asp Ser Thr Tyr Tyr Ala Glu Ser Val
    50                  55                  60
Lys Gly Arg Phe Thr Ile Ser Arg Asp Asn Ala Arg Asn Thr Val Tyr
65                  70                  75                  80
Leu Gln Met Asn Ser Leu Arg Ala Glu Asp Thr Ala Val Tyr Tyr Cys
                85                  90                  95
Ala Ser Gly Ile Leu Thr Gly Tyr Val Phe Asp Tyr Trp Gly Lys Gly
            100                 105                 110
Thr Met Val Thr Val Ser Ser Gly Gly Gly Gly Ser Gly Gly Gly Gly
        115                 120                 125
Ser Gly Gly Gly Gly Ser Asp Val Val Met Thr Gln Ser Pro Leu Ser
    130                 135                 140
Leu Pro Val Thr Leu Gly Gln Pro Ala Ser Ile Ser Cys Arg Ser Ser
145                 150                 155                 160
Gln Ser Leu Val Ser Asn Asp Gly Asn Thr Tyr Leu Asn Trp Phe Gln
                165                 170                 175
Gln Arg Pro Gly Gln Pro Pro Arg Arg Leu Ile Tyr Met Val Ser Lys
            180                 185                 190
Trp Asp Ser Gly Val Pro Asp Arg Phe Ser Gly Ser Gly Ser Gly Thr
        195                 200                 205
Asp Phe Thr Leu Arg Ile Ser Arg Val Glu Ala Glu Asp Val Gly Val
    210                 215                 220
Tyr Tyr Cys Met Gln Ser Thr His Trp Pro Pro Thr Phe Gly Gly Gly
225                 230                 235                 240
Thr Lys Leu Glu Ile Lys Arg
                245

<210>  26
<211>  741
<212>  DNA
<213>  Artificial Sequence

<220>
<223>  Nucleotide sequence of 2H8's scFv

<400>  26
gaggtgcagc tggtggagac tgggggcggc ttggtccagc ctggggggtc cctgagactc      60
tcctgtgcag cgtctggatt caccttcagt agctatggca tgcactgggt ccgccaggct     120
ccagggaagg ggctggagtg ggtctcagtt attagtggta gtggtgatag tacatactac     180
gcagagtccg tgaagggccg cttcaccatc tccagagaca atgccaggaa cacggtgtat     240
ctgcaaatga acagtctgag agccgaggac acggctgtat attattgtgc aagtggcatt     300
ttgactggtt atgtatttga ctattggggc aaagggacaa tggtcaccgt ctcgagtggt     360
ggaggcggtt caggcggagg tggttctggc ggtggcggat cggatgttgt gatgactcag     420
tctccactct ccctgcccgt cacccttgga cagccggcct ccatctcctg caggtctagt     480
caaagcctcg tatccaatga tggaaacacc tacttgaatt ggtttcagca gaggccaggc     540
caacctccaa ggcgcctaat ttatatggtt tctaagtggg actctggggt cccagacaga     600
ttcagcggca gtgggtcagg cactgatttc acactgagaa tcagcagggt ggaggctgag     660
gatgttgggg tttattactg catgcaaagt acacactggc ctcccacttt cggcggaggg     720
accaagctgg agatcaaacg t                                               765

<210>  27
<211>  54
<212>  PRT
<213>  Artificial Sequence

<220>
<223>  E2

<400>  27
Lys Val Arg Pro Asn Glu Glu Asn Asn Lys Asp Ala Asp Leu Tyr Thr
1               5                   10                  15
Ser Arg Val Met Leu Ser Ser Gln Val Pro Leu Glu Pro Pro Leu Leu
            20                  25                  30
Phe Leu Leu Glu Glu Tyr Lys Asn Tyr Leu Asp Ala Ala Asn Met Ser
        35                  40                  45
Met Arg Val Arg Arg His
    50

<210>  28
<211>  36
<212>  PRT
<213>  Artificial Sequence

<220>
<223>  E5

<400>  28
Val Met Leu Ser Ser Gln Val Pro Leu Glu Pro Pro Leu Leu Phe Leu
1               5                   10                  15
Leu Glu Glu Tyr Lys Asn Tyr Leu Asp Ala Ala Asn Met Ser Met Arg
            20                  25                  30
Val Arg Arg His
        35

<210>  29
<211>  12
<212>  PRT
<213>  Artificial Sequence

<220>
<223>  DH

<400>  29
Asp Ala Ala Asn Met Ser Met Arg Val Arg Arg His
1               5                   10

<210>  30
<211>  56
<212>  PRT
<213>  Artificial Sequence

<220>
<223>  E1

<400>  30
Pro Met Lys Glu Ala Asn Ile Arg Gly Gln Gly Gly Leu Ala Tyr Pro
1               5                   10                  15
Gly Val Arg Thr His Gly Thr Leu Glu Ser Val Asn Gly Pro Lys Ala
            20                  25                  30
Gly Ser Arg Gly Leu Thr Ser Leu Ala Asp Thr Phe Glu His Met Ile

35                  40                  45
Glu Glu Leu Leu Asp Glu Asp Gln
    50                  55

<210>  31
<211>  37
<212>  PRT
<213>  Artificial Sequence

<220>
<223>  E3


<400>  31
Pro Met Lys Glu Ala Asn Ile Arg Gly Gln Gly Gly Leu Ala Tyr Pro
1               5                   10                  15
Gly Val Arg Thr His Gly Thr Leu Glu Ser Val Asn Gly Pro Lys Ala
            20                  25                  30
Gly Ser Arg Gly Leu
        35

<210>  32
<211>  37
<212>  PRT
<213>  Artificial Sequence

<220>
<223>  VP



<400>  32
Thr Ser Leu Ala Asp Thr Phe Glu His Met Ile Glu Glu Leu Leu Asp
1               5                   10                  15
Glu Asp Gln Lys Val Arg Pro Asn Glu Glu Asn Asn Lys Asp Ala Asp
            20                  25                  30
Leu Tyr Thr Ser Arg
        35

<210>  33
<211>  12
<212>  PRT
<213>  Artificial Sequence

<220>
<223>  VP



<400>  33
Val Met Leu Ser Ser Gln Val Pro Leu Glu Pro Pro
1               5                   10

<210>  34
<211>  12
<212>  PRT
<213>  Artificial Sequence

<220>
<223>  LL

<400>  34
Leu Leu Phe Leu Leu Glu Glu Tyr Lys Asn Tyr Leu
1               5                   10

<210>  35
<211>  426
<212>  PRT
<213>  Homo sapiens

<220>
<223>  p75NTR

<400>  35
Met Gly Ala Gly Ala Thr Gly Arg Ala Met Asp Gly Pro Arg Leu Leu
1               5                   10                  15
Leu Leu Leu Leu Leu Gly Val Ser Leu Gly Gly Ala Lys Glu Ala Cys
            20                  25                  30
Pro Thr Gly Leu Tyr Thr His Ser Gly Glu Cys Cys Lys Ala Cys Asn
        35                  40                  45
Leu Gly Glu Gly Val Ala Gln Pro Cys Gly Ala Asn Gln Thr Val Cys
    50                  55                  60
Glu Pro Cys Leu Asp Ser Val Thr Phe Ser Asp Val Val Ser Ala Thr
65                  70                  75                  80
Glu Pro Cys Lys Pro Cys Thr Glu Cys Val Gly Leu Gln Ser Met Ser
                85                  90                  95
Ala Pro Cys Val Glu Ala Asp Asp Ala Val Cys Arg Cys Ala Tyr Gly
            100                 105                 110
Tyr Tyr Gln Asp Glu Thr Thr Gly Arg Cys Glu Ala Cys Arg Val Cys
        115                 120                 125
Glu Ala Gly Ser Gly Leu Val Phe Ser Cys Gln Asp Lys Gln Asn Thr
    130                 135                 140
Val Cys Glu Glu Cys Pro Asp Gly Thr Tyr Ser Asp Glu Ala Asn His
145                 150                 155                 160
Val Asp Pro Cys Leu Pro Cys Thr Val Cys Glu Asp Thr Glu Arg Gln
                165                 170                 175
Leu Arg Glu Cys Thr Arg Trp Ala Asp Ala Glu Cys Glu Glu Ile Pro
            180                 185                 190
Gly Arg Trp Ile Thr Arg Ser Thr Pro Pro Glu Gly Ser Asp Ser Thr
        195                 200                 205
Ala Pro Ser Thr Gln Glu Pro Glu Ala Pro Pro Glu Gln Asp Leu Ile
    210                 215                 220
Ala Ser Thr Val Ala Gly Val Val Thr Thr Val Met Gly Ser Ser Gln
225                 230                 235                 240
Pro Val Val Thr Arg Gly Thr Thr Asp Asn Leu Ile Pro Val Tyr Cys
                245                 250                 255
Ser Ile Leu Ala Ala Val Val Val Gly Leu Val Ala Tyr Ile Ala Phe
            260                 265                 270
Lys Arg Trp Asn Ser Cys Lys Gln Asn Lys Gln Gly Ala Ser Arg Pro
        275                 280                 285
Val Asn Gln Thr Pro Pro Pro Glu Gly Glu Lys Leu His Ser Asp Ser
    290                 295                 300
Gly Ile Ser Val Asp Ser Gln Ser Leu His Asp Gln Gln Pro His Thr
305                 310                 315                 320
Gln Thr Ala Ser Gly Gln Ala Leu Lys Gly Asp Gly Gly Leu Tyr Ser
                325                 330                 335
Ser Leu Pro Pro Ala Lys Arg Glu Glu Val Glu Lys Leu Leu Asn Gly
            340                 345                 350
Ser Ala Gly Asp Thr Trp Arg His Leu Ala Gly Glu Leu Gly Tyr Gln
        355                 360                 365
Pro Glu His Ile Asp Ser Phe Thr His Glu Ala Cys Pro Val Arg Ala
    370                 375                 380

Leu Leu Ala Ser Trp Ala Thr Gln Asp Ser Ala Thr Leu Asp Ala Leu
385                 390                 395                 400
Leu Ala Ala Leu Arg Arg Ile Gln Arg Ala Asp Leu Val Glu Ser Leu
                405                 410                 415
Cys Ser Glu Ser Thr Ala Thr Ser Pro Val
            420                 425

<210>  36
<211>  831
<212>  PRT
<213>  Homo sapiens

<220>
<223>  sortilin

<400>  36
   Met Glu Arg Pro Trp Gly Ala Ala Asp Gly Leu Ser Arg Trp Pro His
   1               5                   10                  15
   Gly Leu Gly Leu Leu Leu Leu Leu Gln Leu Leu Pro Pro Ser Thr Leu
               20                  25                  30
   Ser Gln Asp Arg Leu Asp Ala Pro Pro Pro Pro Ala Ala Pro Leu Pro
           35                  40                  45
   Arg Trp Ser Gly Pro Ile Gly Val Ser Trp Gly Leu Arg Ala Ala Ala
       50                  55                  60
   Ala Gly Gly Ala Phe Pro Arg Gly Gly Arg Trp Arg Arg Ser Ala Pro
   65                  70                  75                  80
   Gly Glu Asp Glu Glu Cys Gly Arg Val Arg Asp Phe Val Ala Lys Leu
                   85                  90                  95
   Ala Asn Asn Thr His Gln His Val Phe Asp Asp Leu Arg Gly Ser Val
               100                 105                 110
   Ser Leu Ser Trp Val Gly Asp Ser Thr Gly Val Ile Leu Val Leu Thr
           115                 120                 125
   Thr Phe His Val Pro Leu Val Ile Met Thr Phe Gly Gln Ser Lys Leu
       130                 135                 140
   Tyr Arg Ser Glu Asp Tyr Gly Lys Asn Phe Lys Asp Ile Thr Asp Leu
   145                 150                 155                 160
   Ile Asn Asn Thr Phe Ile Arg Thr Glu Phe Gly Met Ala Ile Gly Pro
                   165                 170                 175
   Glu Asn Ser Gly Lys Val Val Leu Thr Ala Glu Val Ser Gly Gly Ser
               180                 185                 190
   Arg Gly Gly Arg Ile Phe Arg Ser Ser Asp Phe Ala Lys Asn Phe Val
           195                 200                 205
   Gln Thr Asp Leu Pro Phe His Pro Leu Thr Gln Met Met Tyr Ser Pro
       210                 215                 220
   Gln Asn Ser Asp Tyr Leu Leu Ala Leu Ser Thr Glu Asn Gly Leu Trp
   225                 230                 235                 240
   Val Ser Lys Asn Phe Gly Gly Lys Trp Glu Glu Ile His Lys Ala Val
                   245                 250                 255
   Cys Leu Ala Lys Trp Gly Ser Asp Asn Thr Ile Phe Phe Thr Thr Tyr
               260                 265                 270
   Ala Asn Gly Ser Cys Lys Ala Asp Leu Gly Ala Leu Glu Leu Trp Arg
           275                 280                 285
   Thr Ser Asp Leu Gly Lys Ser Phe Lys Thr Ile Gly Val Lys Ile Tyr
       290                 295                 300
   Ser Phe Gly Leu Gly Gly Arg Phe Leu Phe Ala Ser Val Met Ala Asp
   305                 310                 315                 320
   Lys Asp Thr Thr Arg Arg Ile His Val Ser Thr Asp Gln Gly Asp Thr
                   325                 330                 335
   Trp Ser Met Ala Gln Leu Pro Ser Val Gly Gln Glu Gln Phe Tyr Ser
               340                 345                 350
   Ile Leu Ala Ala Asn Asp Asp Met Val Phe Met His Val Asp Glu Pro
   355                 360                 365
   Gly Asp Thr Gly Phe Gly Thr Ile Phe Thr Ser Asp Asp Arg Gly Ile
       370                 375                 380
   Val Tyr Ser Lys Ser Leu Asp Arg His Leu Tyr Thr Thr Thr Gly Gly
   385                 390                 395                 400
   Glu Thr Asp Phe Thr Asn Val Thr Ser Leu Arg Gly Val Tyr Ile Thr
                   405                 410                 415
   Ser Val Leu Ser Glu Asp Asn Ser Ile Gln Thr Met Ile Thr Phe Asp
               420                 425                 430
   Gln Gly Gly Arg Trp Thr His Leu Arg Lys Pro Glu Asn Ser Glu Cys
           435                 440                 445
   Asp Ala Thr Ala Lys Asn Lys Asn Glu Cys Ser Leu His Ile His Ala
       450                 455                 460
   Ser Tyr Ser Ile Ser Gln Lys Leu Asn Val Pro Met Ala Pro Leu Ser
   465                 470                 475                 480
   Glu Pro Asn Ala Val Gly Ile Val Ile Ala His Gly Ser Val Gly Asp
                   485                 490                 495
   Ala Ile Ser Val Met Val Pro Asp Val Tyr Ile Ser Asp Asp Gly Gly
               500                 505                 510
   Tyr Ser Trp Thr Lys Met Leu Glu Gly Pro His Tyr Tyr Thr Ile Leu
           515                 520                 525
   Asp Ser Gly Gly Ile Ile Val Ala Ile Glu His Ser Ser Arg Pro Ile
       530                 535                 540
   Asn Val Ile Lys Phe Ser Thr Asp Glu Gly Gln Cys Trp Gln Thr Tyr
   545                 550                 555                 560
   Thr Phe Thr Arg Asp Pro Ile Tyr Phe Thr Gly Leu Ala Ser Glu Pro
                   565                 570                 575
   Gly Ala Arg Ser Met Asn Ile Ser Ile Trp Gly Phe Thr Glu Ser Phe
               580                 585                 590
   Leu Thr Ser Gln Trp Val Ser Tyr Thr Ile Asp Phe Lys Asp Ile Leu
           595                 600                 605
   Glu Arg Asn Cys Glu Glu Lys Asp Tyr Thr Ile Trp Leu Ala His Ser
       610                 615                 620
   Thr Asp Pro Glu Asp Tyr Glu Asp Gly Cys Ile Leu Gly Tyr Lys Glu
   625                 630                 635                 640
   Gln Phe Leu Arg Leu Arg Lys Ser Ser Val Cys Gln Asn Gly Arg Asp
                   645                 650                 655
   Tyr Val Val Thr Lys Gln Pro Ser Ile Cys Leu Cys Ser Leu Glu Asp
               660                 665                 670
   Phe Leu Cys Asp Phe Gly Tyr Tyr Arg Pro Glu Asn Asp Ser Lys Cys
           675                 680                 685
   Val Glu Gln Pro Glu Leu Lys Gly His Asp Leu Glu Phe Cys Leu Tyr
       690                 695                 700
   Gly Arg Glu Glu His Leu Thr Thr Asn Gly Tyr Arg Lys Ile Pro Gly
   705                 710                 715                 720
   Asp Lys Cys Gln Gly Gly Val Asn Pro Val Arg Glu Val Lys Asp Leu
                   725                 730                 735
   Lys Lys Lys Cys Thr Ser Asn Phe Leu Ser Pro Glu Lys Gln Asn Ser
               740                 745                 750
   Lys Ser Asn Ser Val Pro Ile Ile Leu Ala Ile Val Gly Leu Met Leu
           755                 760                 765
   Val Thr Val Val Ala Gly Val Leu Ile Val Lys Lys Tyr Val Cys Gly
       770                 775                 780
   Gly Arg Phe Leu Val His Arg Tyr Ser Val Leu Gln Gln His Ala Glu
   785                 790                 795                 800
   Ala Asn Gly Val Asp Gly Val Asp Ala Leu Asp Thr Ala Ser His Thr
                   805                 810                 815
   Asn Lys Ser Gly Tyr His Asp Asp Ser Asp Glu Asp Leu Leu Glu
               820                 825                 830

<210>  37
<211>  1159
<212>  PRT

<213>  Homo sapiens

<220>
<223>  SORCS2

<400>  37
Met Ala His Arg Gly Pro Ser Arg Ala Ser Lys Gly Pro Gly Pro Thr
1               5                   10                  15
Ala Arg Ala Pro Ser Pro Gly Ala Pro Pro Pro Pro Arg Ser Pro Arg
            20                  25                  30
Ser Arg Pro Leu Leu Leu Leu Leu Leu Leu Leu Gly Ala Cys Gly Ala
        35                  40                  45
Ala Gly Arg Ser Pro Glu Pro Gly Arg Leu Gly Pro His Ala Gln Leu
    50                  55                  60
Thr Arg Val Pro Arg Ser Pro Pro Ala Gly Arg Ala Glu Pro Gly Gly
65                  70                  75                  80
Gly Glu Asp Arg Gln Ala Arg Gly Thr Glu Pro Gly Ala Pro Gly Pro
                85                  90                  95
Ser Pro Gly Pro Ala Pro Gly Pro Gly Glu Asp Gly Ala Pro Ala Ala
            100                 105                 110
Gly Tyr Arg Arg Trp Glu Arg Ala Ala Pro Leu Ala Gly Val Ala Ser
        115                 120                 125
Arg Ala Gln Val Ser Leu Ile Ser Thr Ser Phe Val Leu Lys Gly Asp
    130                 135                 140
Ala Thr His Asn Gln Ala Met Val His Trp Thr Gly Glu Asn Ser Ser
145                 150                 155                 160
Val Ile Leu Ile Leu Thr Lys Tyr Tyr His Ala Asp Met Gly Lys Val
                165                 170                 175
Leu Glu Ser Ser Leu Trp Arg Ser Ser Asp Phe Gly Thr Ser Tyr Thr
            180                 185                 190
Lys Leu Thr Leu Gln Pro Gly Val Thr Thr Val Ile Asp Asn Phe Tyr
        195                 200                 205
Ile Cys Pro Thr Asn Lys Arg Lys Val Ile Leu Val Ser Ser Ser Leu
    210                 215                 220
Ser Asp Arg Asp Gln Ser Leu Phe Leu Ser Ala Asp Glu Gly Ala Thr
225                 230                 235                 240
Phe Gln Lys Gln Pro Ile Pro Phe Phe Val Glu Thr Leu Ile Phe His
                245                 250                 255
Pro Lys Glu Glu Asp Lys Val Leu Ala Tyr Thr Lys Glu Ser Lys Leu
            260                 265                 270
Tyr Val Ser Ser Asp Leu Gly Lys Lys Trp Thr Leu Leu Gln Glu Arg
        275                 280                 285
Val Thr Lys Asp His Val Phe Trp Ser Val Ser Gly Val Asp Ala Asp
    290                 295                 300
Pro Asp Leu Val His Val Glu Ala Gln Asp Leu Gly Gly Asp Phe Arg
305                 310                 315                 320
Tyr Val Thr Cys Ala Ile His Asn Cys Ser Glu Lys Met Leu Thr Ala
                325                 330                 335
Pro Phe Ala Gly Pro Ile Asp His Gly Ser Leu Thr Val Gln Asp Asp
            340                 345                 350
Tyr Ile Phe Phe Lys Ala Thr Ser Ala Asn Gln Thr Lys Tyr Tyr Val
        355                 360                 365
Ser Tyr Arg Arg Asn Glu Phe Val Leu Met Lys Leu Pro Lys Tyr Ala
    370                 375                 380
Leu Pro Lys Asp Leu Gln Ile Ile Ser Thr Asp Glu Ser Gln Val Phe
385                 390                 395                 400
Val Ala Val Gln Glu Trp Tyr Gln Met Asp Thr Tyr Asn Leu Tyr Gln
                405                 410                 415
Ser Asp Pro Arg Gly Val Arg Tyr Ala Leu Val Leu Gln Asp Val Arg
            420                 425                 430
Ser Ser Arg Gln Ala Glu Glu Ser Val Leu Ile Asp Ile Leu Glu Val

435                 440                 445
Arg Gly Val Lys Gly Val Phe Leu Ala Asn Gln Lys Ile Asp Gly Lys
    450                 455                 460
Val Met Thr Leu Ile Thr Tyr Asn Lys Gly Arg Asp Trp Asp Tyr Leu
465                 470                 475                 480
Arg Pro Pro Ser Met Asp Met Asn Gly Lys Pro Thr Asn Cys Lys Pro
                485                 490                 495
Pro Asp Cys His Leu His Leu His Leu Arg Trp Ala Asp Asn Pro Tyr
            500                 505                 510
Val Ser Gly Thr Val His Thr Lys Asp Thr Ala Pro Gly Leu Ile Met
        515                 520                 525
Gly Ala Gly Asn Leu Gly Ser Gln Leu Val Glu Tyr Lys Glu Glu Met
    530                 535                 540
Tyr Ile Thr Ser Asp Cys Gly His Thr Trp Arg Gln Val Phe Glu Glu
545                 550                 555                 560
Glu His His Ile Leu Tyr Leu Asp His Gly Gly Val Ile Val Ala Ile
                565                 570                 575
Lys Asp Thr Ser Ile Pro Leu Lys Ile Leu Lys Phe Ser Val Asp Glu
            580                 585                 590
Gly Leu Thr Trp Ser Thr His Asn Phe Thr Ser Thr Ser Val Phe Val
        595                 600                 605
Asp Gly Leu Leu Ser Glu Pro Gly Asp Glu Thr Leu Val Met Thr Val
    610                 615                 620
Phe Gly His Ile Ser Phe Arg Ser Asp Trp Glu Leu Val Lys Val Asp
625                 630                 635                 640
Phe Arg Pro Ser Phe Ser Arg Gln Cys Gly Glu Glu Asp Tyr Ser Ser
                645                 650                 655
Trp Glu Leu Ser Asn Leu Gln Gly Asp Arg Cys Ile Met Gly Gln Gln
            660                 665                 670
Arg Ser Phe Arg Lys Arg Lys Ser Thr Ser Trp Cys Ile Lys Gly Arg
        675                 680                 685
Ser Phe Thr Ser Ala Leu Thr Ser Arg Val Cys Glu Cys Arg Asp Ser
    690                 695                 700
Asp Phe Leu Cys Asp Tyr Gly Phe Glu Arg Ser Ser Ser Ser Glu Ser
705                 710                 715                 720
Ser Thr Asn Lys Cys Ser Ala Asn Phe Trp Phe Asn Pro Leu Ser Pro
                725                 730                 735
Pro Asp Asp Cys Ala Leu Gly Gln Thr Tyr Thr Ser Ser Leu Gly Tyr
            740                 745                 750
Arg Lys Val Val Ser Asn Val Cys Glu Gly Gly Val Asp Met Gln Gln
        755                 760                 765
Ser Gln Val Gln Leu Gln Cys Pro Leu Thr Pro Pro Arg Gly Leu Gln
    770                 775                 780
Val Ser Ile Gln Gly Glu Ala Val Ala Val Arg Pro Gly Glu Asp Val
785                 790                 795                 800
Leu Phe Val Val Arg Gln Glu Gln Gly Asp Val Leu Thr Thr Lys Tyr
                805                 810                 815
Gln Val Asp Leu Gly Asp Gly Phe Lys Ala Met Tyr Val Asn Leu Thr
            820                 825                 830
Leu Thr Gly Glu Pro Ile Arg His Arg Tyr Glu Ser Pro Gly Ile Tyr
        835                 840                 845
Arg Val Ser Val Arg Ala Glu Asn Thr Ala Gly His Asp Glu Ala Val
    850                 855                 860
Leu Phe Val Gln Val Asn Ser Pro Leu Gln Ala Leu Tyr Leu Glu Val
865                 870                 875                 880
Val Pro Val Ile Gly Leu Asn Gln Glu Val Asn Leu Thr Ala Val Leu
                885                 890                 895
Leu Pro Leu Asn Pro Asn Leu Thr Val Phe Tyr Trp Trp Ile Gly His
            900                 905                 910
Ser Leu Gln Pro Leu Leu Ser Leu Asp Asn Ser Val Thr Thr Arg Phe
        915                 920                 925
Ser Asp Thr Gly Asp Val Arg Val Thr Val Gln Ala Ala Cys Gly Asn
    930                 935                 940

Ser Val Leu Gln Asp Ser Arg Val Leu Arg Val Leu Asp Gln Phe Gln
945                 950                 955                 960
Val Met Pro Leu Gln Phe Ser Lys Glu Leu Asp Ala Tyr Asn Pro Asn
                965                 970                 975
Thr Pro Glu Trp Arg Glu Asp Val Gly Leu Val Val Thr Arg Leu Leu
            980                 985                 990
Ser Lys Glu Thr Ser Val Pro Gln  Glu Leu Leu Val Thr  Val Val Lys
        995                 1000                 1005
Pro Gly  Leu Pro Thr Leu Ala  Asp Leu Tyr Val Leu  Leu Pro Pro Pro
    1010                 1015                 1020
Arg  Pro Thr Arg Lys Arg  Ser Leu Ser Ser Asp  Lys Arg Leu Ala Ala
1025                 1030                 1035                 1040
Ile Gln Gln Val Leu  Asn Ala Gln Lys Ile  Ser Phe Leu Leu Arg  Gly
                1045                 1050                 1055
Gly Val Arg Val  Leu Val Ala Leu Arg  Asp Thr Gly Thr Gly  Ala Glu
            1060                 1065                 1070
Gln Leu Gly  Gly Gly Gly Gly Tyr  Trp Ala Val Val Val  Leu Phe Val
        1075                 1080                 1085
Ile Gly  Leu Phe Ala Ala Gly  Ala Phe Ile Leu Tyr  Lys Phe Lys Arg
    1090                 1095                 1100
Lys  Arg Pro Gly Arg Thr  Val Tyr Ala Gln Met  His Asn Glu Lys Glu
1105                 1110                 1115                 1120
Gln Glu Met Thr Ser  Pro Val Ser His Ser  Glu Asp Val Gln Gly  Ala
                1125                 1130                 1135
Val Gln Gly Asn  His Ser Gly Val Val  Leu Ser Ile Asn Ser  Arg Glu
            1140                 1145                 1150
Met His Ser  Tyr Leu Val Ser
        1155

<210>  38
<211>  678
<212>  PRT
<213>  Homo sapiens

<220>
<223>  extracellular domain of p75NTR and sortilin

<400>  38
Ser Ala Pro Gly Glu Asp Glu Glu Cys Gly Arg Val Arg Asp Phe Val
1               5                   10                  15
Ala Lys Leu Ala Asn Asn Thr His Gln His Val Phe Asp Asp Leu Arg
            20                  25                  30
Gly Ser Val Ser Leu Ser Trp Val Gly Asp Ser Thr Gly Val Ile Leu
        35                  40                  45
Val Leu Thr Thr Phe His Val Pro Leu Val Ile Met Thr Phe Gly Gln
    50                  55                  60
Ser Lys Leu Tyr Arg Ser Glu Asp Tyr Gly Lys Asn Phe Lys Asp Ile
65                  70                  75                  80
Thr Asp Leu Ile Asn Asn Thr Phe Ile Arg Thr Glu Phe Gly Met Ala
                85                  90                  95
Ile Gly Pro Glu Asn Ser Gly Lys Val Val Leu Thr Ala Glu Val Ser
            100                 105                 110
Gly Gly Ser Arg Gly Gly Arg Ile Phe Arg Ser Ser Asp Phe Ala Lys
        115                 120                 125
Asn Phe Val Gln Thr Asp Leu Pro Phe His Pro Leu Thr Gln Met Met
    130                 135                 140
Tyr Ser Pro Gln Asn Ser Asp Tyr Leu Leu Ala Leu Ser Thr Glu Asn
145                 150                 155                 160
Gly Leu Trp Val Ser Lys Asn Phe Gly Gly Lys Trp Glu Glu Ile His
                165                 170                 175
Lys Ala Val Cys Leu Ala Lys Trp Gly Ser Asp Asn Thr Ile Phe Phe

            180                 185                 190
Thr Thr Tyr Ala Asn Gly Ser Cys Lys Ala Asp Leu Gly Ala Leu Glu
        195                 200                 205
Leu Trp Arg Thr Ser Asp Leu Gly Lys Ser Phe Lys Thr Ile Gly Val
    210                 215                 220
Lys Ile Tyr Ser Phe Gly Leu Gly Gly Arg Phe Leu Phe Ala Ser Val
225                 230                 235                 240
Met Ala Asp Lys Asp Thr Thr Arg Arg Ile His Val Ser Thr Asp Gln
                245                 250                 255
Gly Asp Thr Trp Ser Met Ala Gln Leu Pro Ser Val Gly Gln Glu Gln
            260                 265                 270
Phe Tyr Ser Ile Leu Ala Ala Asn Asp Asp Met Val Phe Met His Val
        275                 280                 285
Asp Glu Pro Gly Asp Thr Gly Phe Gly Thr Ile Phe Thr Ser Asp Asp
    290                 295                 300
Arg Gly Ile Val Tyr Ser Lys Ser Leu Asp Arg His Leu Tyr Thr Thr
305                 310                 315                 320
Thr Gly Gly Glu Thr Asp Phe Thr Asn Val Thr Ser Leu Arg Gly Val
                325                 330                 335
Tyr Ile Thr Ser Val Leu Ser Glu Asp Asn Ser Ile Gln Thr Met Ile
            340                 345                 350
Thr Phe Asp Gln Gly Gly Arg Trp Thr His Leu Arg Lys Pro Glu Asn
        355                 360                 365
Ser Glu Cys Asp Ala Thr Ala Lys Asn Lys Asn Glu Cys Ser Leu His
    370                 375                 380
Ile His Ala Ser Tyr Ser Ile Ser Gln Lys Leu Asn Val Pro Met Ala
385                 390                 395                 400
Pro Leu Ser Glu Pro Asn Ala Val Gly Ile Val Ile Ala His Gly Ser
                405                 410                 415
Val Gly Asp Ala Ile Ser Val Met Val Pro Asp Val Tyr Ile Ser Asp
            420                 425                 430
Asp Gly Gly Tyr Ser Trp Thr Lys Met Leu Glu Gly Pro His Tyr Tyr
        435                 440                 445
Thr Ile Leu Asp Ser Gly Gly Ile Ile Val Ala Ile Glu His Ser Ser
    450                 455                 460
Arg Pro Ile Asn Val Ile Lys Phe Ser Thr Asp Glu Gly Gln Cys Trp
465                 470                 475                 480
Gln Thr Tyr Thr Phe Thr Arg Asp Pro Ile Tyr Phe Thr Gly Leu Ala
                485                 490                 495
Ser Glu Pro Gly Ala Arg Ser Met Asn Ile Ser Ile Trp Gly Phe Thr
            500                 505                 510
Glu Ser Phe Leu Thr Ser Gln Trp Val Ser Tyr Thr Ile Asp Phe Lys
        515                 520                 525
Asp Ile Leu Glu Arg Asn Cys Glu Glu Lys Asp Tyr Thr Ile Trp Leu
    530                 535                 540
Ala His Ser Thr Asp Pro Glu Asp Tyr Glu Asp Gly Cys Ile Leu Gly
545                 550                 555                 560
Tyr Lys Glu Gln Phe Leu Arg Leu Arg Lys Ser Ser Val Cys Gln Asn
                565                 570                 575
Gly Arg Asp Tyr Val Val Thr Lys Gln Pro Ser Ile Cys Leu Cys Ser
            580                 585                 590
Leu Glu Asp Phe Leu Cys Asp Phe Gly Tyr Tyr Arg Pro Glu Asn Asp
        595                 600                 605
Ser Lys Cys Val Glu Gln Pro Glu Leu Lys Gly His Asp Leu Glu Phe
    610                 615                 620
Cys Leu Tyr Gly Arg Glu Glu His Leu Thr Thr Asn Gly Tyr Arg Lys
625                 630                 635                 640
Ile Pro Gly Asp Lys Cys Gln Gly Gly Val Asn Pro Val Arg Glu Val
                645                 650                 655
Lys Asp Leu Lys Lys Lys Cys Thr Ser Asn Phe Leu Ser Pro Glu Lys
            660                 665                 670
Gln Asn Ser Lys Ser Asn
        675

## Description

### FIELD OF THE INVENTION

The present disclosure belongs to the field of treatment of B cell related diseases, and specifically relates to the use of proBDNF regulators in the diagnosis, prevention, treatment or improvement of B cell related diseases.

### BACKGROUND OF THE INVENTION

Brain derived neurotrophic factor (BDNF) is a neurotrophic factor discovered after nerve growth factor. It has a molecular weight of 12.4kDa and is mainly distributed in the central nervous system. It has important functions in regulating survival, differentiation, plasticity of synapses and damage repair of neurons, and the like.

Precursor for brain-derived neurotrophic factor, ProBDNF, is synthesized in the endoplasmic reticulum after transcription and translation of BDNF gene. It is a peptide chain with 247 amino acids in length. Positions 1-18 of the amino acid sequence of ProBDNF are the signal peptide sequence. Two fragments are generated during the secretion process. One fragment is a polypeptide fragment comprising amino acids 19-129 of the sequence(i.e. the precursor domain), known as the proBDNF pro-domain, and the other fragment is positions 130-247 of the amino acid sequence(i.e. the fragment encoded by the mature domain), which is processed to form biologically active mBDNF.

At present, a mass of evidence shows that proBDNF is not only used as an intermediate product of mature BDNF synthesis, but also as a ligand which binds to its high affinity receptor p75 neurotrophin receptor (p75NTR, SEQ ID NO: 35), sortilin (SEQ ID NO: 36), SORCS2 (SEQ ID NO: 37) to play a biological role. ProBDNF binds to the extracellular domain of p75NTR and sortilin (SEQ ID NO: 38), forming a complex, and transducing several signals comprising RhoA, JNK and NFkB. There're researches showing that the precursors for neurotrophic factors (including proNGF and proBDNF, etc.) promote cell apoptosis and inflammatory response, but the role of proBDNF and its signals in B cell-related diseases remains unclear.

### SUMMARY OF THE INVENTION

### The problem to be solved by the invention

Based on the existing problems in the prior art, it is necessary to provide a reagent capable of inhibiting or activating the activity of proBDNF or the proBDNF signaling pathway for the preparation of regulators for regulating the function of B lymphocytes. At the same time, there is also a need to provide an effective method for pre-detecting related diseases caused by B lymphocyte dysfunction, and an effective method for predicting the treatment or prevention effect of treating or preventing B lymphocyte dysfunction related diseases.

### Solutions to the problem

In a technical solution, the present disclosure relates to the use of an agent for inhibiting or activating the activity of proBDNF or proBDNF signaling pathway in preparing a regulator for regulating B lymphocyte function, wherein the proBDNF signaling pathway is selected from the group consisting of p75NTR signaling pathway, sortinlin signaling pathway, or SORCS2 signaling pathway.

In a technical solution, the sequence of proBDNF described in the present disclosure is the sequence shown in SEQ ID NO: 1, or a sequence having at least 80% identity with SEQ ID NO: 1; preferably, the sequence of proBDNF has at least 90% identity with SEQ ID NO: 1, more preferably, the sequence of proBDNF has at least 95% identity with SEQ ID NO: 1, and most preferably, the sequence of proBDNF has at least 99% identity with SEQ ID NO: 1.

In a technical solution, the use of the regulator of the present disclosure comprises one or more of the following uses:
(1) regulating B cell activation, proliferation and antigen presentation;
(2) reduceing antibody production;
(3) inhibiting plasmablast differentiation or cytokine production.

In a technical solution, the regulator described in the present disclosure is selected from the group consisting of proBDNF antagonist, p75NTR antagonist, Sortilin antagonist or SORCS2 antagonist. In a technical solution, the use of the antagonist described in the present disclosure comprises one or more of the following uses:
(1) inhibiting or reducing the expression of proBDNF;
(2) interfering with, blocking or preventing the interaction or binding of proBDNF with p75NTR, sortilin or SORCS2;
(3) inhibiting or reducing the expression of p75NTR;
(4) interfering with, blocking or preventing the interaction or binding of p75NTR with proBDNF or sortilin.

In a technical solution, the proBDNF antagonist described in the present disclosure comprises an antibody or protein fragment, siRNA or small molecule compound against proBDNF.

In a technical solution, the p75NTR antagonist described in the present disclosure comprises an antibody against p75NTR, a p75NTR Fc fragment, a p75NTR ECD fragment and/or a small molecule compound.

In a technical solution, the proBDNF antagonist described in the present disclosure specifically recognizes the precursor domain of proBDNF.

In a technical solution, the sequence of the precursor domain of proBDNF described in the present disclosure has the sequence shown in SEQ ID NO: 2, or has at least 80% identity with SEQ ID NO: 2, preferably at least 90% identity, more preferably at least 95% identity, and most preferably at least 99% identity.

In a technical solution, the proBDNF antagonist described in the present disclosure specifically recognizes the sequence shown in SEQ ID NO: 27, 28 or 29, or specifically recognizes a sequence having at least 80% identity, preferably at least 90% identity, more preferably at least 95% identity, and most preferably at least 99% identity with the sequence shown in SEQ ID NO: 27, 28 or 29.

In a technical solution, the proBDNF antagonist described in the present disclosure is an antibody.

In a technical solution, the antibody described in the present disclosure is selected from the group consisting of Fab, Fab', F(ab')₂, Fv, single chain antibody, scFv, diabody, dsFv, single domain antibody, and/or peptide at least partially comprising a complementary determining region.

In a technical solution, the antibody described in the present disclosure is selected from the group consisting of:
antibody 1: HCDR1, HCDR2, and HCDR3 of the heavy chain have the sequences shown in SEQ ID NOs: 3, 4, and 5 respectively, or have the sequences shown in SEQ ID NOs: 13, 14, and 15 respectively;
antibody 2: LCDR1, LCDR2, and LCDR3 of the light chain have the sequences shown in SEQ ID NO: 6, 7, and 8 respectively, or have the sequences shown in SEQ ID NO: 16, 17, and 18 respectively;
antibody 3: having the heavy chain CDR region of antibody 1 and the light chain CDR region of antibody 2;
antibody 4: HCDR1, HCDR2 and HCDR3 of the heavy chain have the sequences shown in SEQ ID NOs: 3, 4 and 5 respectively, and LCDR1, LCDR2 and LCDR3 of the light chain have the sequences shown in SEQ ID NOs: 6, 7, and 8 respectively;
antibody 5: HCDR1, HCDR2 and HCDR3 of the heavy chain have the sequences shown in SEQ ID NOs: 13, 14, and 15 respectively, and LCDR1, LCDR2 and LCDR3 of the light chain have the sequences shown in SEQ ID NOs: 16, 17, and 18 respectively.

In a technical solution, the antibody has:
the heavy chain variable region shown in SEQ ID NO: 9 and the light chain variable region shown in SEQ ID NO: 10; or
the heavy chain variable region shown in SEQ ID NO: 19 and the light chain variable region shown in SEQ ID NO: 20.

In a technical solution, the antibody described in the present disclosure is:
an antibody with human immunoglobulin Fc region, formed by fusing the sequences of the heavy chain variable region shown in SEQ ID NO: 9 and the light chain variable region shown in SEQ ID NO: 10 with one or more heavy chain constant regions; or
an antibody with human immunoglobulin Fc region, formed by fusing the sequences of the heavy chain variable region shown in SEQ ID NO: 19 and the light chain variable region shown in SEQ ID NO: 20 with one or more heavy chain constant regions.

In a technical solution, the antibody described in the present disclosure is a monoclonal antibody, a murine antibody, a chimeric antibody, a humanized antibody, or a fully human antibody.

In another technical solution, the present disclosure also relates to the use of a regulator in the preparation of a medicament for treating B lymphocyte dysfunction-related diseases, wherein it treats the diseases by the following method, comprising administering to a subject a therapeutically effective amount of the regulators.

In another technical solution, the B lymphocyte dysfunction-related diseases are selected from the group consisting of B lymphocyte tumor, infectious disease, atherosclerosis, premature birth, body fluid rejection of transplant patients, graft-versus-host disease (GVHD) of transplant recipients, post-transplant lymphoproliferative disease.

In another technical solution, the B lymphocyte tumor is selected from the group consisting of chronic lymphocytic leukemia (CLL), small lymphocytic lymphoma (SLL), B cell prolymphocytic leukemia (B-PLL), non-CLL/SLL lymphoma, mantle cell lymphoma, multiple myeloma, Waldenstrom's macroglobulinemia, or a combination thereof.

In another technical solution, the B lymphocyte is selected from the group consisting of a circulating B lymphocyte, a blood B lymphocyte, a splenic B lymphocyte, a marginal zone B lymphocyte, a follicular B lymphocyte, a peritoneal B lymphocyte and/or a bone marrow B lymphocyte.

In another technical solution, when treating or improving the B lymphocyte tumor, the regulator and other anti-tumor drugs are administered simultaneously or sequentially.

In another technical solution, it prevents, treats, or improves the atherosclerosis, premature birth, body fluid rejection of transplant patients, and graft-versus-host disease (GVHD) of transplant recipients or post-transplant lymphoproliferative disease, while simultaneously administering the regulator and other immune antagonists.

In another technical solution, the present disclosure also relates to a method for predicting the therapeutic or preventive effect of treating or preventing B lymphocyte dysfunction-related diseases, comprising the following steps:
(1) detecting the expression level of proBDNF in a subject;
(2) administing the regulator according to any one of claims 1-16 to the subject;
(3) detecting the change in the expression level of proBDNF in the subject relative to (1) after the administration of the regulator shown in (2).

In another technical solution, the present disclosure also relates to the use of an antibody in the preparation of a reagent or kit for detecting/treating B lymphocyte dysfunction-related diseases, wherein the antibody is selected from the group consisting of proBDNF antibody, p75NTR antibody, sortinlin antibody or SORCS2 antibody.

### Effect of the invention

In a technical solution, the present disclosure demonstrates the function of proBDNF in B cells, and for the first time proves that proBDNF regulators cause B cell activation, proliferation and antigen presentation, reduce antibody production, or inhibit plasmablast differentiation and cytokine production.

In a technical solution, the present disclosure provides a reagent that inhibits or activates the activity of proBDNF or the proBDNF signaling pathway. The aforementioned reagent comprises monoclonal antibodies that regulates B cell function and isuseful in treating B lymphocyte dysfunction-related diseases. The aforementioned proBDNF signaling pathway is selected from the group consisting of p75NTR signaling pathway, sortinlin signaling pathway, or SORCS2 signaling pathway. Wherein, the aforementioned monoclonal antibody is selected from the group consisting of 1D3 and 2H8, and has a good affinity for human proBDNF.

In a technical solution, the present disclosure provides a reagent or kit, which is useful in effective pre-detection of related diseases caused by B lymphocyte dysfunction.

In a technical solution, the present disclosure provides an effective method for predicting the therapeutic or preventive effects of treating or preventing B lymphocyte dysfunction-related diseases.

### BRIEF DESCRIPTION OF THE DRAWINGS

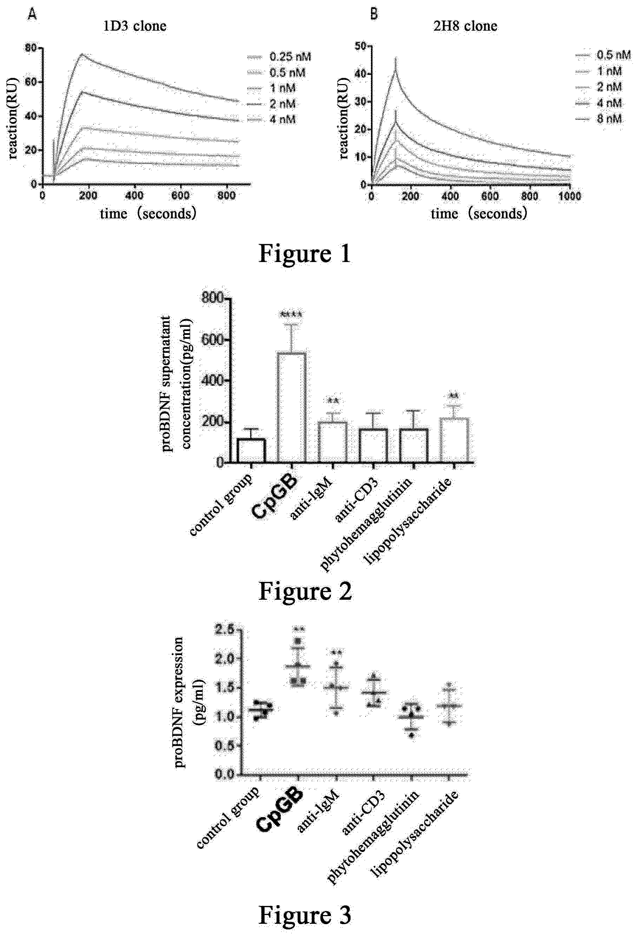

A and B in Figure 1 show the kinetic curves of monoclonal antibodies 1D3 and 2H8, respectively, in the Biacore affinity determination experiment.
Figure 2 shows the concentration of proBDNF in the supernatant after B cell activation.
Figures 3A and 3B show the the expression of proBDNF (A) and mBDNF (B) in PBMC cells under different conditions detected by using Elisa.
Figures 4A and 4B show the effects of different agonists on the expression of proBDNF (A) and mBDNF (B) in PBMCs detected by Western blot.
Figure 5 shows the flow cytometric fluorescence intensity of CD40 and HLA-DR on the surface of CD19+B cells after CpGB treatment; wherein, the two columns of the IgG group and the two columns of the proBDNF group in A and B in Figure 5 are the relative fluorescence intensity, the left column is normal saline, and the right column is CpGB.
A in Figure 6 and B in Figure 6 show the effect of CpGB treatment on B cell proliferation; C in Figure 6 shows a flow cytometry graph of CSFE stained B cells.
Figure 7 shows the effect of proBDNF antibody on CpGB-induced antibody production by B cells in in PBMCs.
Figure 8 shows the effect of proBDNF antibody on CpGB promoting B cell differentiation.
Figure 9 shows the ELISA results of 1D3 and 2H8 with polypeptides E1-E5.
Figure 10 shows the ELISA results of 1D3 and 2H8 with polypeptide DH.
Figure 11 shows the fluorescence intensity of p75(CD271) in B cells stimulated by CpGB, Anti-IgM, PHA and LPS.

### DETAIL DESCRIPTION OF THE INVENTION

The present disclosure relates to the application of pro-BDNF regulators in B cell-related diseases. To facilitate the understanding of the present disclosure, some terms are defined as follows.

When used in conjunction with the term "comprising" in the claims and/or specification, the word "a" or "an" can mean "one", but can also mean "one or more", "at least one" and "one or more than one".

As used in the claims and specification, the words "comprise", "have", "include" or "contain" mean inclusive or open-ended, and do not exclude additional, unquoted elements or method steps. Although the disclosed content supports the definition of the term "or" only as an alternative and "and/or", unless expressly stated that it's only an alternative or that the alternatives are mutually exclusive, the term "or" in the claims means "and/or".

When used in the claims or specification, the selected/optional/preferred "numerical range" comprises both the value range endpoints at both ends of the range and all natural numbers covered between the numerical endpoints relative to the foregoing numerical endpoints. For example, the "sequence having at least 80% identity" described in the present disclosure refers to the sequence having 80%, 81%, 82%, 83%, 84%, 85%, 86%, 87%, 88%, 89%, 90%, 91%, 92%, 93%, 94%, 95%, 96%, 97%, 98%, 99%, 100% identity relative to the standard sequence.

The term "proBDNF" (precursor for brain-derived neurotrophic factor) is "precursor of brain-derived neurotrophic factor", which is the precursor product of BDNF gene, and the sequence defined in SEQ ID NO: 1 is the nucleic acid sequence of human pro-BDNF. Positions 1-18 of the amino acid sequence of proBDNF are the signal peptide sequence. Two fragments are generated at this site during the secretion process, one of which is a polypeptide fragment comprising amino acids 19-129 (i.e. the precursor domain) of the sequence, which is refered to as proBDNF pro-domain, and the sequence defined in SEQ ID NO: 2 is the nucleic acid sequence of the precursor domain of human pro-BDNF; the other fragment is positions 130-247 of the amino acid sequence(i.e. the fragment encoded by the mature domain), which is processed to form mature BDNF with biological activity. The term "p75NTR" is the neurotrophin receptor (P75 neurotrophin receptor, p75NTR), also known as CD271, which is the ligand of proBDNF, and may have the same meaning as P75 herein. p75NTR is a low-affinity receptor for neurotrophic factors, belonging to the members of the tumor necrosis factor receptor (TNFR) superfamily, and the earliest known NT (neurotrophin) receptor to be isolated. It is mainly expressed abundantly during the early development of nerve cells, and through different signaling pathways, it induces neurocyte dominated cell proliferation, migration, differentiation, survival, apoptosis, synapse establishment and nerve formation to exert multiple biological effects. At present, a large amount of evidence shows that proBDNF not only acts as an intermediate product of mature BDNF synthesis, but also acts as a ligand which binds to its high-affinity receptor p75NTR to exert biological effects.

The term "Sortilin" is a sorting protein, which belongs to the prototype members of the Vps10p-domain receptor family. It can also be refered to as neurotensin receptor 3 (NTR3), glycoprotein 95 (GP95) or 100kDa NT receptor. Sorting proteins are type I membrane receptors expressed in many tissues comprising brain, spinal cord, testis, liver and skeletal muscle (Petersen, Nielsen et al. 1997; Hermans-Borgmeyer, Hermey et al. 1999).

The term "SORCS2" stands for receptor for vacuolar protein sorting protein, another member of the receptor family of vacuolar protein sorting protein domain. SORCS2 has 1150 amino acids and is a type I transmembrane glycoprotein receptor. It is highly expressed in the brain and kidneys. proBDNF can combine with SORCS2 to form a proBDNF-p75NTR-SORCS2 complex.

The term "B lymphocyte", or B cell for short, is a pluripotent stem cell derived from bone marrow. The differentiation process of mammalian B cells can be mainlydivided into five stages: pre-B cells, immature B cells, mature B cells, activated B cells and plasmocytes. The differentiation of pre-B cells and immature B cells is antigen independent, and the differentiation process is carried out in the bone marrow. The antigen-dependent stage refers to that mature B cells are activated after antigen stimulation and continue to differentiate into plasmocytes that synthesize and secrete antibodies. The differentiation at this stage is mainly carried out in the peripheral immune organs. Plasmocytes can synthesize and secrete antibodies and circulate in the blood. B-cell lymphoma is the most common lymphocytic leukemia. Stem cells or pre-B cells from the bone marrow, after immigrating into the bursa of fabricius or bursa of fabricius-like organs, gradually differentiate into B cells with immune potential. Mature B cells migrate out through the peripheral blood and enter the spleen and lymph nodes. They are mainly distributed in the spleen nodules, splenic cord and lymph nodules, lymphatic cords and submucosal lymph nodes of the digestive tract. After being stimulated by antigens, they differentiate and proliferate into plasmocytes, synthesizing antibodies to exert humoral immunity function. There are more B cells in bone marrow and collecting lymph nodes than T cells, less B cells in blood and lymph nodes than T cells, and even fewer in thoracic ducts, and only a few participate in recirculation. There are many different markers on the cell membrane of B cells, mainly surface antigens and surface receptors. These surface markers are giant protein molecules bound to the cell membrane. B1 cells are T cell independent cells. B2 is a T cell dependent cell. Survival time of B cells *in* the body is short, only a few days to a few weeks, but the memory cells of B cells can exist in the body for a long time. B lymphocyte dysfunction related diseases comprise B lymphocyte tumors, infectious diseases, atherosclerosis and premature birth.

The term "antagonist" refers to a class of substances that can bind to receptors but have no intrinsic activity. An antagonist is opposite to the term "agonist". After binding to a receptor, an antagonist cannot induce a conformational change whichinduces biological activity change. The term "agonist" is capable of inducing a conformational change of the receptor to trigger biological activity. The term "p75NTR antagonist" as used in the present disclosure refers to a molecule that inhibits the expression level of components in the proBDNF-p75NTR ligand-receptor system in B cells (also referred to as "an expression antagonist"); or, that inhibits the interaction or binding between components of the proBDNF-p75NTR ligand-receptor system expressed in B cells (also called "a binding antagonist"), thereby reducing the amount, formation, and function of the ligand-receptor system, and/or its downstream signals.

The term "proBDNF antagonist" as used in the present disclosure can inhibit B cell activation, proliferation, antigen presentation, reduce antibody production, and/or inhibit plasmablast differentiation, cytokine production, etc. If a certain molecule causes a significant decrease in the expression of a component (transcription or translation level), it is considered that the molecule inhibits the expression level of the system component. Similarly, if certain molecules cause a significant reduction in the binding between the component and the formed ligand-receptor complex, it results in a significant reduction in downstream signals and functions mediated by the ligand-receptor system, such as reduction in antibody production, it is considered that the molecule inhibits the binding between components of the ligand-receptor system.

Binding antagonists can work in two ways. Binding antagonists can compete with the ligand proBDNF for receptors, thereby interfering with, blocking or preventing the binding of proBDNF to p75NTR, sortilin and/or SORCS2. This type of antagonist, which binds to the receptor without triggering the expected signal transduction, is also refered to as a "competitive antagonist", which can comprise, for example, an oligopeptide designed based on the proBDNF sequence, or an antibody against SORCS2. Alternatively, the binding antagonist can bind to and isolate the ligand proBDNF, which has sufficient affinity and specificity with the ligand, and can substantially interfere with, block or prevent the binding of proBDNF to p75NTR, sortilin and/or SORCS2. This type of antagonist is also referred to as a "neutralizing antagonist", which may comprise, for example, an antibody or aptamer directed against proBDNF, which specifically binds to proBDNF.

The characteristics of an antagonist can also be determined based on the target molecule to be antagonized by the antagonist. For example, a proBDNF antagonist refers to a molecule that inhibits or reduces the expression of proBDNF, or interferes with, blocks, or prevents the interaction or binding of proBDNF with p75NTR, sortilin, and/or SORCS2. On the other hand, a p75NTR antagonist refers to a molecule that inhibits or reduces the expression of p75NTR, or interferes with, blocks, or prevents the interaction of p75NTR with proBDNF and/or sortilin.

The term "protein fragment" refers to immunoglobulin molecules and immunoreactive parts of immune molecules, that is, molecules that comprise an antigen binding site which specifically binds to an antigen ("immune response"). The term "protein fragment" also comprises immunoglobulin molecules derived from various species, comprising invertebrates and vertebrates. Structurally, the simplest naturally occurring antibody (e.g., IgG) comprises four polypeptide chains: two heavy (H) chains and two light chain (L) chains interconnected by disulfide bonds. Immunoglobulins represent a large family of molecules comprising several types of molecules, such as IgD, IgG, IgA, IgM, and IgE. The term "protein fragment" comprises, for example, hybrid antibodies or modified antibodies and fragments thereof. It has been shown that the antigen-binding function of antibodies can be performed by fragments of naturally occurring antibodies. These fragments are collectively referred to as "antigen binding units". The term "protein fragment" also comprises any polypeptide chain containing molecular structuresthat have specific shapes that matches the epitopes and recognizes the epitopes, wherein one or more non-covalent binding interactions stabilize the complex between the molecular structure and the epitope. Examples of the antigen binding unit comprise Fab fragments, monovalent fragments consisted of VL, VH, CL and CH1 domains, and bivalent fragments comprising two Fab fragments connected by a disulfide bridge on the hinge region (F(ab)2 fragments); Fd fragments consisted of VH and CH1 domains, Fv fragments consisted of single-arm VL and VH domains of antibodies; dAb fragments consisted of VH domains (Ward et al., Nature, 341:544-546, 1989); and an isolated complementary determining region (CDR) or any fusion protein comprising such antigen binding units.

As used herein, the term "antibody" refers to the full-length or one or more fragments of an antibody that retains the ability to specifically bind to an antigen (e.g., a part of pro-BDNF), or other polypeptides that have similar antibody binding activity. Naturally occurring "antibodies" are glycoproteins comprising at least two heavy (H) chains and two light (L) chains connected by interchain disulfide bonds. Each heavy chain is consisted of a heavy chain variable region (abbreviated as VH herein) and a heavy chain constant region. The heavy chain constant region is consisted of three domains CH1, CH2 and CH3. Each light chain is consisted of a light chain variable region (abbreviated as VL herein) and a light chain constant region. The light chain constant region consists of a domain CL. The VH and VL regions can be further subdivided into regions of high variability, refered to as complementary determining regions (CDR), separated by more conservative regions called framework regions (ER). Each VH and VL is consisted of 3 CDRs and 4 FRs arranged in the following order: FR1, CDR1, FR2, CDR2, FR3, CDR3, FR4 from the amino terminal to the carboxy terminal. The variable regions of the heavy and light chains comprise binding domains that interact with antigens. The constant region of an antibody can mediate the binding of immunoglobulins to host tissues or factors (comprising various cells of the immune system (such as effector cells) and the first component (C1q) of the classical complement system).

It has been shown that the antigen-binding function of antibodies can be performed by fragments of full-length antibodies. An antibody fragment refers to a partial region of the aforementioned intact antibody (such as a monoclonal antibody or a polyclonal antibody), for example, Fab, Fab', F(ab')2, Fv (antibody variable region), single chain antibodies (H chain, L chain, V region of H chain, V region of L chain, etc.), scFv, diabody (i.e., scFv dimer), dsFv (disulfide stabilized V region), and peptides at least partially comprising a complementary determining regions (CDRs), etc.

The terms "complementary determining region" and "CDR" refer to the amino acid sequence that confer antigen specificity and binding affinity in the variable region of an antibody. Generally, there are 3 CDRs (HCDR1, HCDR2, HCDR3) in each heavy chain variable region and 3 CDRs (LCDR1, LCDR2, LCDR3) in the light chain variable region.

The amino acid sequence boundaries of a given CDR can be determined using any of many well-known plans, comprising those described by Kabat et al. (1991), "Sequences of Proteins of Immunological Interest," 5th edition, Public Health Service, National Institutes of Health, Bethesda, MD ("Kabat" numbering plan), Al-Lazikani et al. (1997) JMB 273, 927-948 ("Chothia" numbering plan).

Although the two domains VL and VH of the Fv fragment are encoded by separate genes, they can be joined using recombination methods through synthetic linkers that allow them to be produced as a single chain protein in which the VL and VH regions are paired (scFv) (see, for example, Bird et al. 1988, Science 242:423-426; and Huston et al. 1988, Proc. Natl. Acad. Sci. 85:5879-5883). Such single chain antibodies are also intended to be comprised in the term "antigen-binding fragment" of antibodies. These antibody fragments are obtained using conventional techniques known to those of ordinary skill in the art, and the functions of the fragments can be screened in the same manner as used for intact antibodies.

The term "single domain antibody" cloned from camel-derived heavy chain antibody (HCAb), a single chain antibody consisting of only one heavy chain variable region. Its size is only 2.4×4nm. It is the smallest fragment capable of binding antigen, refered to as a single domain antibody (VHH) or nanobody.

The term "epitope" means a protein determinant capable of specifically binding an antibody. Epitopes usually consist of chemically active surface groups of molecules such as amino acids or sugar side chains, and usually have specific structural characteristics and charge characteristics.

The term "fully human antibody" is intended to comprise antibodies having variable regions in which the framework and CDR regions are derived from sequences of human origin. In addition, if the antibody comprises a constant region, then the constant region is also derived from such human sequences, for example, human germline sequences, or mutated versions of antibodies of human germline sequences or common framework sequences from human framework sequences analysis (e.g., as described in Knappik et al., 2000, J Mol Biol 296: 57-86). The fully human antibodies of the present disclosure comprise amino acid residues not encoded by human sequences (e.g., mutations introduced by random or site-directed mutagenesis in vitro or by somatic mutation in vivo). However, as used herein, the term "fully human antibody" is not intended to comprise antibodies in which CDR sequences derived from the germline of another mammalian species, such as a mouse, have been grafted onto human framework sequences.

The term "recombinant human antibody" comprises all human antibodies prepared, expressed, produced or isolated by recombinant means. Such as antibodies isolated from transgenic or transchromosomic animals (such as mice) of human immunoglobulin genes or hybridomas prepared therefrom, antibodies isolated from host cells transformed to express human antibodies, e.g., from transfected tumors, antibodies isolated from recombinant combinatorial human antibody library, and antibodies prepared, expressed, produced, or isolated by any other means comprising splicing all or part of human immunoglobulin gene sequences with other DNA sequences. Such recombinant human antibodies have variable regions in which both the framework and CDR regions are derived from human germline immunoglobulin sequences. However, in certain embodiments, such recombinant human antibodies can be subjected to in vitro mutagenesis (or, when transgenic animals with human Ig sequences are used, somatic mutagenesis in vivo), thereby although the recombinant amino acid sequences in the VH and VL regions are derived from human germline VH and VL sequences and are related to the sequences, they may not naturally exist in the human antibody germline repertoire in vivo.

The term "isotype" refers to the type of antibodies provided by the heavy chain constant region gene (e.g., IgM, IgE, IgG, such as IgG1 or IgG4).

Since the antigen binding specificity of an antibody is mainly provided by the CDR1, 2 and 3 regions, the VH CDR1, 2 and 3 sequences can be "mixed and matched" with the VL CDR1, 2 and 3 sequences (i.e., CDRs from different antibodies can be mixed and matched, and each antibody comprising VH CDR 1, 2 and 3 and VL CDR 1, 2 and 3 is generated as an other anti-pro-BDNF binding molecule of the present disclosure). The pro-BDNF binding of such "mixed and matched" antibodies can be tested using the binding assays described above and in the examples or other conventional assays (e.g., ELISA). When mixing and matching VH CDR sequences, structurally similar CDR sequences should be used to replace the CDR1, CDR2 and/or CDR3 sequences from a specific VH sequence. Likewise, when mixing and matching VL CDR sequences, structurally similar CDR sequences should be used to replace the CDR1, CDR2, and/or CDR3 sequences from a specific VL sequence. It is obvious to those of ordinary skill in the art that the novel VH and VH can be generated by substituting one or more VH and/or VL CDR region sequences with structurally similar sequences from the CDR sequences of the monoclonal antibodies of the present disclosure shown herein.

As used herein, if the variable region chain or full-length chain of an antibody is obtained from a system using human germline immunoglobulin genes, the human antibody comprises products that are specific germline sequences or heavy or light chain variable regions or full-length heavy or light chains derived therefrom. Such systems comprise immunizing transgenic mice with human immunoglobulin genes with the target antigen, or screening a library of human immunoglobulin genes displayed on phage with the target antigen. Human antibodies that are "products of" or "derived from" human germline immunoglobulin sequences are identified by comparing the amino acid sequences of the human antibodies with the amino acid sequences of human germline immunoglobulins and selecting the human germline immunoglobulin sequences that are closest in sequence to the sequences of the human antibodies (i.e., the greatest percentage identity). Human antibodies that are "products of" or "derived from" a specific human germline immunoglobulin sequence may comprise amino acids that differ from the germline sequence due to, for example, the intentional introduction of naturally occurring somatic mutations or site-specific mutations. However, the selected human antibody usually has at least 90% identity with the amino acid sequence encoded by the human germline immunoglobulin gene in amino acid sequence, and comprises amino acid residues that identify human antibodies as human when compared with the amino acid sequences of germline immunoglobulins of other species (e.g., murine germline sequences). In certain cases, the human antibody has at least 60%, 70%, 80%, 90%, or at least 95% or even at least 96%, 97%, 98% or 99% identity in amino acid sequence with the amino acid sequence encoded by germline immunoglobulin genes. Generally, a human antibody derived from a particular human germline sequence will show a difference of no more than 10 amino acids from the amino acid sequence encoded by the human germline immunoglobulin gene. In certain cases, human antibodies show a difference of no more than 5 or even no more than 4, 3, 2, or 1 amino acid from the amino acid sequence encoded by the germline immunoglobulin gene.

### Antibodies with conservative modifications

In certain embodiments, the antibody of the present disclosure has a heavy chain variable region comprising HCDR1, HCDR2, and HCDR3 sequences and a light chain variable region comprising LCDR1, LCDR2, and LCDR3 sequences, wherein one or more of these CDR sequences have designated amino acid sequences based on the antibody 1D3 or 2H8 described herein or the conservative modifications thereof, and wherein the antibody or protein retains the desired functional properties of the anti-pro-BDNF antibody of the present disclosure.

As used herein, the term "conservative sequence modification" is intended to mean an amino acid substitution in which an amino acid residue is replaced by an amino acid residue with a similar side chain. Families of amino acid residues with similar side chains have been defined in the art. These families comprise amino acids with basic side chains (for example, lysine, arginine, histidine), amino acids with acidic side chains (for example, aspartic acid, glutamic acid), and amino acids with uncharged polar side chains (for example, glycine, asparagine, glutamine, serine, threonine, tyrosine, cysteine, tryptophan), amino acids with non-polar side chains (for example, alanine, valine, leucine, isoleucine, proline, phenylalanine, methionine), amino acids with β-branched side chains (for example, threonine, valine, isoleucine) and amino acids with aromatic side chains (for example, tyrosine, phenylalanine, tryptophan, histidine). Therefore, one or more amino acid residues in the CDR region of the antibody of the present disclosure can be replaced with other amino acid residues from the same side chain families, and the functional assay described herein can be used to test the retained function of the altered antibody.

Modifications can be introduced into the antibodies disclosed herein by standard techniques known in the art such as site-directed mutagenesis and PCR-mediated mutagenesis.

In addition, in addition to antibodies 1D3 and 2H8, the present disclosure also covers homologous antibodies or proteins that retain the ideal functional properties of 1D3 and 2H8 antibodies.

At the same time, the use of a "therapeutically effective amount" of the anti-pro-BDNF antibody or protein described in the present disclosure can lead to a reduction in the severity of disease symptoms, an increase in the frequency and duration of the asymptomatic period, or the prevention of damage or disability caused by the disease.

The composition of the present disclosure can be administered via one or more routes of administration using one or more of a variety of methods known in the art. As those of ordinary skill in the art will understand, the route and/or mode of administration will vary depending on the desired result. Routes of administration of antibodies of the present disclosure comprise intravenous, intramuscular, intradermal, intraperitoneal, subcutaneous, spinal, or other parenteral routes of administration, such as by injection or infusion. As used herein, the phrase "parenteral administration" means administration modes other than enteral and topical administration, usually by injection, comprising but not limited to intravenous, intramuscular, intraarterial, intrathecal, intrasaccular, intraorbital, intracardiac, intradermal, intraperitoneal, transtracheal, subcutaneous, subepidermal, intraarticular, subcapsular, subarachnoid, intraspine, epidural and intrasternal injections and infusions.

Alternatively, the antibodies or proteins of the present disclosure can be administered via non-parenteral routes, e.g., topical, epidermal or mucosal administration routes, such as intranasal, oral, vaginal, rectal, sublingual, or topical.

The antibodies or proteins of the present disclosure can be prepared with carriers that will protect the antibodies from rapid release, such as controlled release formulations, comprising implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods used to prepare such dosage forms have been patented or well known to those skilled in the art. See, for example, Sustained and Controlled Release Drug Delivery Systems, J. R. Robinson, Editor, Marcel Dekker Inc., New York, 1978.

The therapeutic composition can be administered by medical means known in the art. For example, in one embodiment, the therapeutic composition of the present disclosure can be administered by needle-free subcutaneous injection, such as those disclosed in US 5,399,163; US 5,383,851; US5,312,335, and the like.

Examples of well-known implants and modules that can be used in the present disclosure comprise: US 4,487,603, which discloses an implantable micro-infusion pump for dispensing drugs at a controlled rate; US 4,486,194, which discloses a therapeutic device for administering drugs through the skin; US4,447,233, which discloses a drug infusion pump for delivering drugs at a precise infusion rate; US4,447,224, which shows a variable flow implantable infusion device for continuous drug delivery; US4,439,196, which discloses an osmotic drug delivery system with multi-chamber compartments; US4,475,196, which discloses an osmotic drug delivery system. Many other implants, delivery systems, and modules of such type are known to those of ordinary skill in the art.

In certain embodiments, the antibodies or proteins of the present disclosure can be formulated to ensure proper distribution in the body. For example, the blood-brain barrier (BBB) excludes many highly hydrophilic compounds. To ensure that the therapeutic compounds of the present disclosure to pass the BBB (if necessary), they can be formulated in, for example, liposomes. For the method of manufacturing liposomes, see, for example, US 4,522,811, US 5,374,548 and 5,399,331. The liposome may comprise one or more moieties that are selectively transported to specific cells or organs, thereby enhancing targeted drug delivery (see, for example, V.V. Ranade 1989, J. Cline Pharmacol. 29:685). Exemplary targeting moieties comprise folate or biotin (see, for example, U.S. Patent No. 5,416,016 belonging to Low et al.), mannosides (Umezawa et al. 1988, Biochem. Biophys. Res. Commun. 153:1038); antibodies (P.G.Bloeman et al. 1995, FEBS Lett. 357:140; M. Owais et al. 1995, Antimicrob. Agents Chernother. 39:180); Surfactant protein A receptor (Briscoe et al. 1995, Am. J. Physiol. 1233:134); p120 (Schreier et al. 1994, J. Biol. Chem. 269:9090); see also Keinanen and Laukkanen 1994, FEBSLett. 346:123; Killion and Fidler 1994, Immunomethods 4:273.

### Uses and methods of this disclosure

The antibodies or proteins of the present disclosure have diagnostic and therapeutic functions in vitro and in vivo. For example, these molecules can be administered to cells in culture (e.g., in vitro or in vivo) or in a subject (e.g., in vivo) to treat, prevent, or diagnose B cell related disorders.

The method is particularly suitable for the treatment, prevention or diagnosis of B cell malignancies, atherosclerosis, premature birth, autoimmune disorders, body fluid rejection of transplant patients, and graft-versus-host disease (GVHD) of transplant recipients and post-transplant lymphoproliferative disease.

Specifically, B cell malignancies comprise chronic lymphocytic leukemia (CLL), small lymphocytic lymphoma (SLL), B cell prolymphocytic leukemia (B-PLL), non-CLL/SLL lymphoma, mantle cell Lymphoma, multiple myeloma, Waldenstrom's macroglobulinemia, or a combination thereof.

In addition, B cell related disorders can also comprise multiple sclerosis, asthma, arthritis, or psoriasis, diabetes, etc. Such diseases also comprise allergies and allergic conditions, hypersensitivity reactions, chronic obstructive pulmonary disease, cystic fibrosis, and organ or tissue transplant rejection.

In addition, when regulators targeting proBDNF are used to treat, prevent and improve B cell related diseases, regulators of proBDNF can also be administered as the only active ingredient or in combination with other drugs e.g., immunosuppressants, immunoregulators, or other cytotoxic or anticancer agents (for example, as an adjuvant or in combination with them), for example, to treat or prevent the aforementioned diseases. For example, the proBDNF regulator can be used in combination with the following drugs: DMARD, e.g., gold salt, sulfasalazine, antimalarial drugs, methotrexate, D-penicillamine, azathioprine, mycophenolic acid, tacrolimus, sirolimus, minocycline, Leflunomide, glucocorticoids; calcineurin inhibitors, e.g., cyclosporin A or FK 506; regulators of lymphocyte recycling, e.g., FTY720 and FTY720 analogs; mTOR inhibitors, e.g., rapamycin 40-O-(2-hydroxyethyl)-rapamycin, CCI779, ABT578, AP23573 or TAFA-93; ascomycins with immunosuppressive properties, e.g., ABT-281, ASM981, etc.; corticosteroids; cyclophosphamide; Azathioprine; Leflunomide; Mizoribine; Mycophenolate mofetil; 15-deoxyspergualin or its immunosuppressive homologs, analogs or derivatives; immunosuppressive monoclonal antibodies, e.g., monoclonal antibodies directed against leukocyte receptors, such as MHC, CD2, CD3, CD4, CD7, CD8, CD25, CD28, CD40, CD45, CD58, CD80, CD86 or their ligands; others immunoregulatory compounds, such as a recombinant binding molecule having at least a part of the extracellular domain of CTLA4 or its mutants, e.g. at least a part of the extracellular domain of CTLA4 or its mutants which linked to non-CTLA4 protein sequence, e.g., CTLA4Ig (e.g., designated as ATCC 68629) or its mutants, such as LEA29Y; adhesion molecule inhibitors (e.g., LFA-1 antagonists, ICAM-1 or -3 antagonists, VCAM-4 antagonists or VLA-4 antagonists); or chemotherapy agents (e.g., paclitaxel, gemcitabine, cisplatin, adriamycin or 5-fluorouracil; anti-TNF drugs, such as monoclonal antibodies against TNF (e.g. infliximab, adalimumab, CDP870), or receptor constructs against TNF-RI or TNF-RII (e.g. etanercept, PEG-TNF-RI); pro-inflammatory cytokine blockers, IL1 blockers, such as anakinra or IL1 traps, canakinumab, IL13 blockers, IL4 blockers, IL6 blockers, IL17 blockers (e.g. secukinumab, broadalumab, ixekizumab); chemokine blockers (e.g. inhibitors or activators of proteases such as metalloproteinases), anti-IL15 antibodies, anti-IL6 antibodies, anti-IL4 antibodies, anti-IL13 antibodies, anti-CD20 antibodies, NSAIDs, such as aspirin or anti-infective agents.

The present disclosure will be further explained below in conjunction with specific examples. It should be understood that these examples are only used to illustrate the present disclosure and not to limit the scope of the present disclosure. The experimental methods that do not specify specific conditions in the following examples are usually according to conventional conditions such as those described in J. Sambrook et al., Molecular Cloning Experiment Guide, 3rd Edition, Science Press, 2002, or according to the conditions suggested by the manufacturer.

### Example 1. Screening of single chain antibodies for the human proBDNF precursor

Phage display technology was used to screen human proBDNF precursor protein specific antibodies from a fully human natural antibody library. For this purpose, the conventional phage display method in the art was adopted to prepare a phage display library, and four rounds of directed screening were carried out for the biotin-labeled human proBDNF precursor recombinant protein. Conventional operations in the art was adopted in the preparation method and display method of the phage display library, as disclosed in PCT/CN2016/096292, Antibody Phage Display: methods and protocols (edited by Robert Aitken, 2009).

Ninety-six clones were selected from the screened clones, and their ability to bind human proBDNF precursor was analyzed by single phage ELISA (enzyme-linked immunosorbent assay). Combined with sequencing analysis, It was observed that single chain antibody 1D3 (see SEQ ID NO: 24 for nucleotide sequence, SEQ ID NO: 23 for amino acid sequence) and 2H8 (see SEQ ID NO: 26 for nucleotide sequence, see SEQ ID NO: 25 for amino acid sequence), have strong binding signals to human proBDNF precursor protein in ELISA experiment.

1D3 and 2H8 protein monoclonal antibodies were prepared, and recombinant monoclonal antibodies 1D3 and 2H8 were obtain after purification.

The affinity and kinetic parameters of the 1D3 and 2H8 monoclonal antibodies were measured by the capture method using the Biacore T200 system (purchased from GE). The operation is referred to the instruction. The adopted concentrations of human proBDNF precursor protein were 0.25nM, 0.5nM, 1nM, 2nM, 4nM and 8nM, respectively. The Biacore T200 evaluation software was used to evaluate the obtained action curve and to calculate the KD value of affinity . Figure 1A and Figure 1B respectively show the kinetic curves of monoclonal antibodies 1D3 and 2H8 in the Biacore affinity determination experiment.

The binding data of 1D3 and 2H8 monoclonal antibodies to human proBDNF precursor protein were summarized in Table 1.

**Table 1. Affinity parameters of monoclonal antibodies of 1D3 and 2H8 to human proBDNF precursor protein**

| Antibody sample | ka (1/Ms) | kd (1/s) | KD (M) |
|---|---|---|---|
| 1D3 Monoclonal antibody | 5.256E+06 | 7.994E-04 | 1.521E-10 |
| 2H8 Monoclonal antibody | 3.103E+06 | 5.940E-03 | 1.914E-09 |

### Example 2. Secretion of proBDNF in PBMCs stimulated under different conditions

PBMCs were derived from the buffy coat of the peripheral blood of normal blood donors. PBMCs were isolated and counted according to conventional techniques in the art. The cells were diluted with complete RPMI medium to 2^{∗}10⁶/ml, and 250ul cell suspension (500,000 cells per well) was added to each well of a 96-well plate, and cultured in a constant temperature incubator at 37°C and 5% CO2 for 72h after adding different stimuli. The grouping and stimulus usage were as follows:
Normal control group (NT): no stimulus was added;
CpGB group (B cell agonist): 2.5ul 100X CpG ODN B (Genework) was add, the working concentration was 3.2ug/ml
Anti-IgM group (B cell agonist): 2.5ul 100X anti-human IgM BCR cross-link antibody (Jackson ImmunoResearch) was add, the working concentration was 10ug/ml;
Anti-CD3 group (T cell activator): 1ul Dynabeads® Human T-Activator CD3/CD28 (Thermol fisher scientific) was add, the working concentration of magnetic beads: cells=1:1;
Phytohemagglutinin (PHA, T cell activator): 2.5ul 100X Phytohemagglutinin (PHA, Sigma) was add, the working concentration was 10ug/ml. The ELISA detection of proBDNF was performed by the operation known in the art (such as J Neurochem. 2015), and the Graphpad prism 6.0 software was adopted for the analysis, and the measurement data was shown as mean ± standard error. One-way analysis of variance was adopted for intergroup analysis, and pairwise comparisons within groups were analyzed by adopting Tukey or Bonferroni method. P<0.05 was considered as statistically significant.

The results showed (Figure 2): The release of proBDNF was significantly increased after stimulation by CpGB and anti-IgM; while the effect of anti-CD3 and PHA on the release of proBDNF was not obvious. These results indicated that B cell activators (CpGB and Anti-IgM) could promote PBMCs to secrete proBDNF. The T cell activator had no such effect, indicating that after activation, B cell caused the secretion of proBDNF.

### Example 3. ProBDNF Expression of PBMCs by Stimulation under Different Conditions

The isolation and culturing of PBMCs was referred to Example 2, and the ELISA analysis is refer to the operation of Example 2 . The results showed (Figures 3A and 3B) that the expression of proBDNF in PBMCs was significantly up-regulated under CpGB and Anti-IgM stimulation (Figure 3A); after CpGB, Anti-IgM, and PHA treatment, mBDNF in PBMCs was significantly down-regulated (Figure 3B), suggesting B Cell agonists can increase the expression of proBDNF in PBMCs.

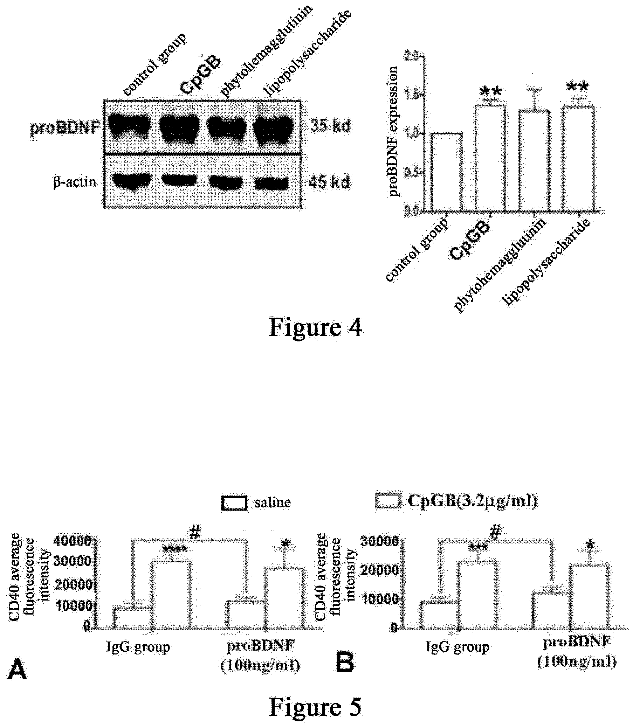

Western Blot analysis was adopted, and conventional technical means by those skilled in the art were adopted in the analysis method. The cell lysate was obtained by conventional operations in the art, and then the corresponding volume of loading buffer (Loading Buffer, Bio-RAD, Lot#161-0747) and 1/20 1M DTT were added to each tube at a ratio of 1:3, and heated in a heater at 98°C for 10 minutes, and after cooling, 1/20 1M DTT was added again and then the lysate was subjected to Western Blot analysis. Anti-proBDNF primary antibody: fully human monoclonal antibody 1D3 was adopted, the working concentration was 1:1000; anti-mBDNF primary antibody: santa cruz, sc-546; the working concentration was 1:1000. The results were shown in Figure 4, CpGB and PHA caused the up-regulation of proBDNF expression in PMBCs.

Both ELISA and Western Blot methods proved that activating B cells can lead to the up-regulation of proBDNF expression in PBMCs, indicating that B cells were the main source of proBDNF expression and secretion.

### Example 4. The effect of proBDNF on B cell activation

After counting PBMCs, the cells were diluted with complete RPMI medium to 2^{∗}10⁶/ml, and 250ul cell suspension (500,000 cells per well) was added to each well of a 96-well plate, and cultured in a constant temperature incubator at 37°C and 5% CO2 for 72 hours after adding different stimuli. The grouping and stimulus usage were as follows:
Normal control group (NT): no stimulus was added;
proBDNF group: 2.5ul 100X recombinant proBDNF protein was added, the working concentration was 100ng/ml;
CpGB group: 2.5ul 100X CpG ODN B (Genework) was added, the working concentration was 3.2ug/ml;
CpGB+proBDNF group: CpGB and proBDNF stimuli were added simultaneously.
Cell surface molecules were analysis by flow cytometry. The specific steps were the same as in Example 11. The combinations of fluorescence and antibodies on cell surface were as follows: CD19: FITC⁺; CD40: PE-Cy5⁺; HLA-DR: BV510.

The experimental results were shown in Figure 5. After CpGB treatment, the flow fluorescence intensity of CD19⁺B cell surface molecules, CD40 and HLA-DR, were significantly higher than those of the untreated group. Exogenous proBDNF protein treatment (proB) promoteed the activation of resting B cells and up-regulateed the expression of CD40 and HLA-DR on the surface. These results indicate that proBDNF can activate B cells.

### Example 5. Effect of clone 1D3 on the proliferation of B cells and T cells in PBMCs

After counting PBMCs, the cells were diluted with complete RPMI medium to 2^{∗}10⁶/ml, and 250ul cell suspension (500,000 cells per well) was added to each well of a 96-well plate after staining the cells with CFSE, and cultured in a constant temperature incubator at 37°C and 5% CO2 for 72 hours after adding different stimuli. The grouping and stimulus usage were as follows:
Normal control group (NT): no stimulus was added;
proBDNF group: 2.5ul 100X recombinant proBDNF protein was added every other day, working concentration was 50ng/ml;
Ab-proBDNF group: 2.5ul 100X Ab-proBDNF (1D3) was added, working concentration was 2ug/ml;
CpGB group: 2.5ul 100X CpG ODN B (Genework) was added, working concentration was 3.2ug/ml; cells=1:1;
CpGB+proBDNF group: CpGB and proBDNF stimuli were added simultaneously;
CpGB+Ab-proBDNF group: CpGB and 1D3 stimuli were added simultaneously;
IgG isotype control group: CpGB/anti-CD3 and IgG isotype control (eBioscience, 2ug/ml) were added respectively.

The CSFE method was adopted to detect the proliferation of B cells. Flow cytometry was used for the analysis of cell surface molecules. The combination of fluorescence and antibody on cell surface was as follows: CD19: FITC⁺.

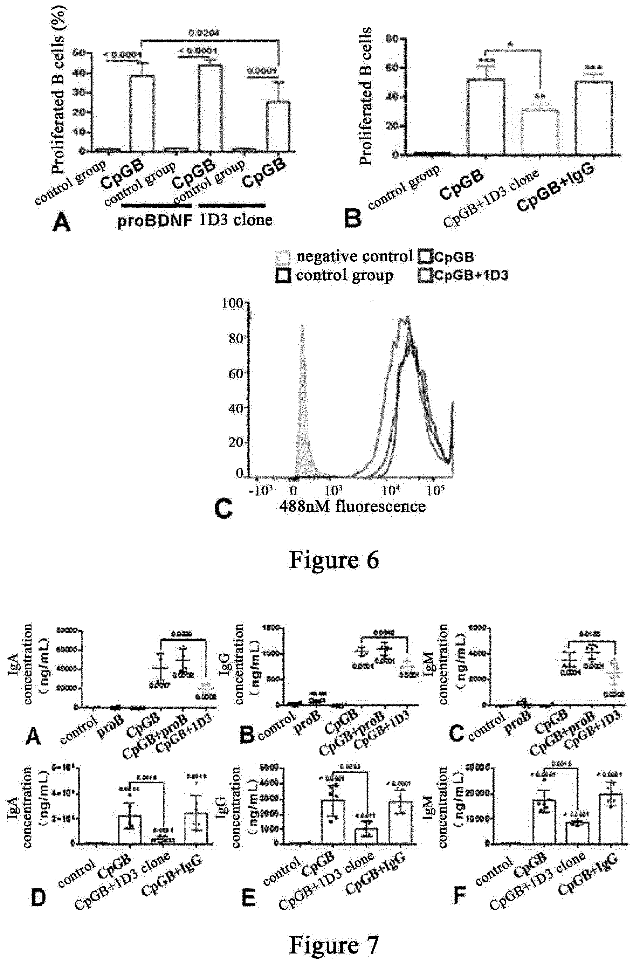

The statistical results were shown in Figures 6A and 6B, and Figure 6C shows the flow cytometry of CSFE stained B cells; the effect of CpGB treatment on B cell proliferation. Compared with the untreated group (NT group), CD19⁺B cells proliferated significantly after CpGB treatment. Compared with the CpGB group, the B cell proliferation of the CpGB+proBDNF group was significantly increased; the CpGB+Ab-proBDNF (1D3) group had a significant decrease in the degree of B cell proliferation compared with the CpGB group. These results indicate that the clone 1D3 monoclonal antibody can significantly inhibit the proliferation of B cells caused by CpGB treatment.

These results indicate that the clone 1D3 significantly inhibits the proliferation of B cells.

### Example 6. Effect of clone 1D3 on CpGB-induced antibody production by B cells in PBMCs

After counting PBMCs, the cells were diluted with complete RPMI medium to 2^{∗}10⁶/ml, and 250ul cell suspension (500,000 cells per well) was added to each well of a 96-well plate, and cultured in a constant temperature incubator at 37°C and 5% CO2 for 10 days after adding different stimuli. The grouping and stimulus usage were as follows:
Normal control group (NT): no stimulus was added;
proBDNF group: 2.5ul 100X recombinant proBDNF protein was added every other day, working concentration was 50ng/ml;
Ab-proBDNF group: 2.5ul 100X Ab-proBDNF (clone 1D3) was added, working concentration was 2ug/ml;
CpGB group: 2.5ul 100X CpG ODN B (Genework) was added, working concentration was 3.2ug/ml;
CpGB+proBDNF group: CpGB and proBDNF stimuli were added simultaneously;
CpGB+Ab-proBDNF group: CpGB and Ab-proBDNF (clone 1D3) stimuli were added simultaneously;
IgG isotype control group: CpGB/anti-CD3 and IgG isotype control (eBioscience, 2ug/ml) were added respectively.

The supernatant of PBMCs was taken for the ELISA detection and analysis of IgA/IgG/IgM , and Prism6 was adopted to analyze the changes in the corresponding proBDNF and mBDNF protein content. The experimental results showed (Figure 7) the protein content of IgA, IgG and IgM in the culture supernatant of PBMCs after CpGB stimulation was significantly higher than that of the unstimulated group (Figure 7, red). The intervention of exogenous proBDNF slightly promoted the secretion of IgG in B cells (Figure 7B). Ab-proBDNF (1D3) significantly inhibited the release of IgA, IgG and IgM induced by CpGB, which was manifested as a significant decrease in the secretion of these antibodies in the supernatant (Figure 7, green).

These results indicate that clone 1D3 Ab-proBDNF significantly inhibits the secretion of various antibodies after B cell activation, and further illustrate that Ab-proBDNF inhibits the function of B cells to secrete antibodies.

### Example 7. The effect of clone 1D3 on CpGB-promoted differentiation of B cells into CD27⁺CD38⁺ plasmablasts

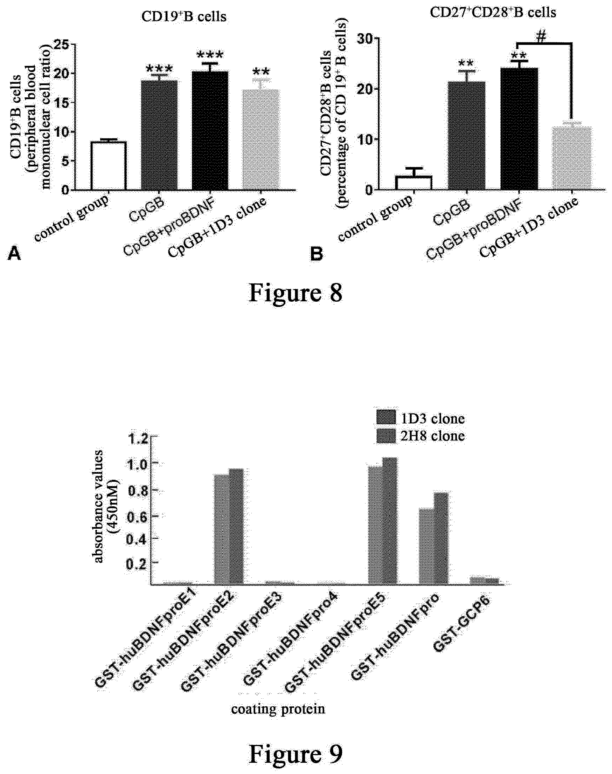

After counting PBMCs, the cells were diluted with complete RPMI medium to 2^{∗}10⁶/ml, and 250ul cell suspension (500,000 cells per well) was added to each well of a 96-well plate, and cultured in a constant temperature incubator at 37°C and 5% CO2 for 10 days after adding different stimuli. The experiment was grouped as follows:
Normal control group (NT): no stimulus was added;
CpGB group: 2.5ul 100X CpG ODN B (Genework) was add, working concentration was 3.2ug/ml;
CpGB+proBDNF group: CpGB and proBDNF stimuli were added simultaneously;
CpGB+Ab-proBDNF group: CpGB and Ab-proBDNF (clone 1D3) stimuli were added simultaneously;
Cell surface molecules were analysis by flow cytometry, combinations of fluorescence and antibodies on cell surface were as follows: CD19: FITC+; CD27: BV421+; CD38: PE-Cy5. The experimental results were shown in Figure 8, wherein the percentage of the number of CD27⁺CD38⁺ plasmablasts (relative to the total number of CD19⁺ B cells) increased significantly, indicating that CpGB can promote the differentiation of B cells into plasmablasts. Exogenous proBDNF slightly increased the number of CD19⁺B cells and CD27⁺CD38⁺ plasmablasts induced by CpGB treatment, but it was not statistically significant. In the CpGB+Ab-proBDNF group, the percentage of CD27⁺CD38⁺ plasmablasts in CD19+B cells was significantly lower than that in the CpGB group or CpGB+proBDNF group.

These results indicate that the clone 1D3 Ab-proBDNF antibody can inhibit the differentiation of B cells and plasmablasts, further indicating that the clone 1D3 Ab-proBDNF regulates B cell functions at multiple levels.

### Example 8. Epitope binding experiment of antibody

The results of ELISA showed that antibodies 1D3 and 2H8 binded to denatured human proBDNF, suggesting that the binding epitopes of the two antibodies were likely to be linear. Human proBDNF was equally divided into two peptides (E1, E2) or three peptides (E3, E4, E5), the amino acid sequence was as follows:
E1: pmkeanirgqgglaypgvrthgtlesvngpkagsrgltsladtfehmieelldedq(SEQ ID NO: 30)
E2: kvrpneennkdadlytsrvmlssqvpleppllflleeyknyldaanmsmrvrrh(SEQ ID NO: 27)
E3: pmkeanirgqgglaypgvrthgtlesvngpkagsrgl(SEQ ID NO: 31)
E4: tsladtfehmieelldedqkvrpneennkdadlytsr(SEQ ID NO: 32)
E5: vmlssqvpleppllflleeyknyldaanmsmrvrrh(SEQ ID NO: 28)

The five peptides were fused with GST and expressed, and then purified. The binding of antibodies 1D3 and 2H8 was detected by ELISA. The results were shown in Figure 9. Antibodies 1D3 and 2H8 bind all of full length GST-human proBDNF, GST-human proBDNF-E2 peptide, and GST-human proBDNF-E5 peptide, suggesting that the binding epitopes of these two antibodies were located within these 36 amino acids of the E5 peptide.

### Example 9. Epitope binding experiment of antibody

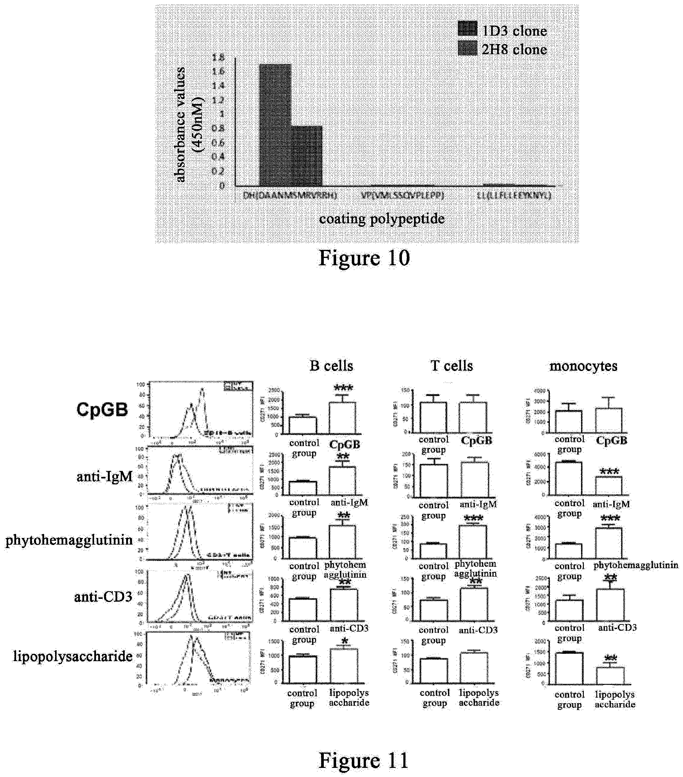

The E5 peptide was further divided into three equal peptide segments, each comprising 12 amino acids, namely VP peptide (VMLSSQVPLEPP, SEQ ID NO: 33), LL peptide (LLFLLEEYKNYL, SEQ ID NO: 34), DH Peptide (DAANMSMRVRRH, SEQ ID NO: 35). Biotinylated VP peptide, LL peptide and DH peptide were synthesized *in vitro* by GL Biochemical (Shanghai) Ltd. and the binding of antibodies was detected by ELISA. The results were shown in Figure 10. The binding of antibody 1D3 to DH peptide was strong, the binding of 2H8 to DH peptide was weak, and there's no binding to the other two peptides, suggesting that the epitope of antibody 1D3 was in the DH peptide, and the epitope of 2H8 was near the DH peptide.

### Example 10. Expression and release of p75NTR in PBMCs (the ligand was expressed on the surface of B cells)

PBMCs were isolated from peripheral blood, and cell surface molecules were analyzed by flow cytometry (BD FACSCanto II). The operation was refered to the product instruction. In addition to the samples to be tested, various control groups were set up. The treatments of each group were as follows:
Blank control group: 100µl of PBS comprising 5% normal human serum was added;
Isotype control group: 100µl of PBS comprising 5µl IgG isotype control (BD Pharmingen, Lot#555748) and 5% normal human serum was added;
Fluorescence compensation control group: 100 µl PBS comprising only one of the antibodies (CD3:PE-cy7 or CD19: FITC or CD14: APC-Cy7 or CD271: PE) and 5% normal human serum was added to each tube;
Group of samples to be tested: 100 µl of PBS comprising cell surface antibody mixture comprising various fluorescent color combinations (CD3:PE-cy7+CD19: FITC+CD14: APC-Cy7+CD271: PE) and 5% normal human serum was added;
The experimental results were shown in Figure 11: the fluorescence intensity of p75(CD271) of was significantly higher than that of the normal non-stimulated control group when B cells were stimulated by CpGB, Anti-IgM, PHA and LPS; p75 on the cell surface was significantly up-regulated when T cells were stimulated by anti-CD3 or PHA, but did not change significantly under other stimuli. These results indicate that different agonists can cause up-regulation of p75 expression on the surface of B cells after activating PBMCs, suggesting that B cells is an important target of proBDNF.

The above-mentioned examples of the present disclosure are merely examples to clearly illustrate the present disclosure, and are not intended to limit the embodiments of the present disclosure. For those of ordinary skill in the art, other changes or modifications in different forms can be made on the basis of the above description. It is unnecessary and impossible to list all the embodiments here. Any modification, equivalent replacement and improvement made within the spirit and principle of this disclosure shall be comprised in the protection scope of the claims of this disclosure.

## Claims

1. Use of an agent for inhibiting or activating the activity of proBDNF or proBDNF signaling pathway in preparing a regulator for regulating B lymphocyte function, wherein the proBDNF signaling pathway is selected from the group consisting of p75NTR signaling pathway, sortinlin signaling pathway, or SORCS2 signaling pathway.

2. The use according to claim 1, wherein the sequence of proBDNF is the sequence shown in SEQ ID NO: 1, or a sequence having at least 80% identity with SEQ ID NO: 1; preferably, the sequence of proBDNF has at least 90% identity with SEQ ID NO: 1, more preferably, the sequence of proBDNF has at least 95% identity with SEQ ID NO: 1, and most preferably, the sequence of proBDNF has at least 99% identity with SEQ ID NO: 1.

3. The use according to any one of claims 1-2, wherein the use of the regulator comprises one or more of the following uses:
(1) regulating B cell activation, proliferation and antigen presentation;
(2) reduceing antibody production;
(3) inhibiting plasmablast differentiation or cytokine production.

4. The use according to any one of claims 1-3, wherein the regulator is selected from the group consisting of proBDNF antagonist, p75NTR antagonist, Sortilin antagonist or SORCS2 antagonist.

5. The use according to claim 4, wherein the use of the antagonist comprises one or more of the following uses:
(1) inhibiting or reducing the expression of proBDNF;
(2) interfering with, blocking or preventing the interaction or binding of proBDNF with p75NTR, sortilin or SORCS2;
(3) inhibiting or reducing the expression of p75NTR;
(4) interfering with, blocking or preventing the interaction or binding of p75NTR with proBDNF or sortilin.

6. The use according to any one of claims 4-5, wherein the proBDNF antagonist comprises an antibody or protein fragment, siRNA or small molecule compound against proBDNF.

7. The use according to any one of claims 4-5, wherein the p75NTR antagonist comprises an antibody against p75NTR, a p75NTR Fc fragment, a p75NTR ECD fragment and/or a small molecule compound.

8. The use according to any one of claims 4-6, wherein the proBDNF antagonist specifically recognizes the precursor domain of proBDNF.

9. The use according to claim 8, wherein the sequence of the precursor domain of proBDNF has the sequence shown in SEQ ID NO: 2, or has at least 80% identity with SEQ ID NO: 2, preferably at least 90% identity, more preferably at least 95% identity, and most preferably at least 99% identity.

10. The use according to claim 9, wherein the proBDNF antagonist specifically recognizes the sequence shown in SEQ ID NO: 27, 28 or 29, or specifically recognizes a sequence having at least 80% identity, preferably at least 90% identity, more preferably at least 95% identity, and most preferably at least 99% identity with the sequence shown in SEQ ID NO: 27, 28 or 29.

11. The use according to any one of claims 4-6, 8-10, wherein the proBDNF antagonist is an antibody.

12. The use according to claim 11, wherein the antibody is selected from the group consisting of Fab, Fab', F(ab')₂, Fv, single chain antibody, scFv, diabody, dsFv, single domain antibody, and/or peptide at least partially comprising a complementary determining region.

13. The use according to claim 11 or 12, wherein the antibody is selected from the group consisting of:
antibody 1: HCDR1, HCDR2, and HCDR3 of the heavy chain have the sequences shown in SEQ ID NOs: 3, 4, and 5 respectively, or have the sequences shown in SEQ ID NOs: 13, 14, and 15 respectively;
antibody 2: LCDR1, LCDR2, and LCDR3 of the light chain have the sequences shown in SEQ ID NO: 6, 7, and 8 respectively, or have the sequences shown in SEQ ID NO: 16, 17, and 18 respectively;
antibody 3: having the heavy chain CDR region of the antibody 1 and the light chain CDR region of the antibody 2;
antibody 4: HCDR1, HCDR2 and HCDR3 of the heavy chain have the sequences shown in SEQ ID NOs: 3, 4 and 5 respectively, and LCDR1, LCDR2 and LCDR3 of the light chain have the sequences shown in SEQ ID NOs: 6, 7, and 8 respectively;
antibody 5: HCDR1, HCDR2 and HCDR3 of the heavy chain have the sequences shown in SEQ ID NOs: 13, 14, and 15 respectively, and LCDR1, LCDR2 and LCDR3 of the light chain have the sequences shown in SEQ ID NOs: 16, 17, and 18 respectively.

14. The use according to claim 13, wherein the antibody has:
the heavy chain variable region shown in SEQ ID NO: 9 and the light chain variable region shown in SEQ ID NO: 10; or
the heavy chain variable region shown in SEQ ID NO: 19 and the light chain variable region shown in SEQ ID NO: 20.

15. The use according to claim 13, wherein the antibody is:
an antibody with human immunoglobulin Fc region, formed by fusing the sequences of the heavy chain variable region shown in SEQ ID NO: 9 and the light chain variable region shown in SEQ ID NO: 10 with one or more heavy chain constant regions; or
an antibody with human immunoglobulin Fc region, formed by fusing the sequences of the heavy chain variable region shown in SEQ ID NO: 19 and the light chain variable region shown in SEQ ID NO: 20 with one or more heavy chain constant regions.

16. The use according to any one of claims 11-15, wherein the antibody is a monoclonal antibody, a murine antibody, a chimeric antibody, a humanized antibody, or a fully human antibody.

17. Use of a regulator in the preparation of a medicament for treating B lymphocyte dysfunction-related diseases, wherein it treats the diseases by the following method, comprising administering to a subject a therapeutically effective amount of the regulators according to any one of claims 1-16.

18. The use according to claim 17, wherein the B lymphocyte dysfunction-related diseases are selected from the group consisting of B lymphocyte tumor, infectious disease, atherosclerosis, premature birth, body fluid rejection of transplant patients, graft-versus-host disease (GVHD) of transplant recipients, post-transplant lymphoproliferative disease.

19. The use according to claim 18, wherein the B lymphocyte tumor is selected from the group consisting of chronic lymphocytic leukemia (CLL), small lymphocytic lymphoma (SLL), B cell prolymphocytic leukemia (B-PLL), non-CLL/SLL lymphoma, mantle cell lymphoma, multiple myeloma, Waldenstrom's macroglobulinemia, or a combination thereof.

20. The use according to claim 17, wherein the B lymphocyte is selected from the group consisting of a circulating B lymphocyte, a blood B lymphocyte, a splenic B lymphocyte, a marginal zone B lymphocytes, a follicular B lymphocyte, a peritoneal B lymphocytes and/or a bone marrow B lymphocyte.

21. The use according to any one of claims 18-20, wherein when treating or improving the B lymphocyte tumor, the regulator and other anti-tumor drugs are administered simultaneously or sequentially.

22. The use according to any one of claims 18-20, wherein it prevents, treats, or improves the atherosclerosis, premature birth, body fluid rejection of transplant patients, and graft-versus-host disease (GVHD) of transplant recipients or post-transplant lymphoproliferative disease, while simultaneously administering the regulator and other immune antagonists.

23. A method for predicting the therapeutic or preventive effect of treating or preventing B lymphocyte dysfunction-related diseases, comprising the following steps:
(1) detecting the expression level of proBDNF in a subject;
(2) administing the regulator according to any one of claims 1-16 to the subject;
(3) detecting the change in the expression level of proBDNF in the subject relative to (1) after the administration of the regulator shown in (2).

24. Use of an antibody in the preparation of a reagent or kit for detecting/treating B lymphocyte dysfunction-related diseases, wherein the antibody is selected from the group consisting of proBDNF
antibody, p75NTR antibody, sortinlin antibody or SORCS2 antibody.
